(19) Europäisches Patentamt / European Patent Office / Office européen des brevets

(11) **EP 4 759 803 A1**

(12) **EUROPEAN PATENT APPLICATION**


(43) Date of publication:
**17.06.2026 Bulletin 2026/25**

(21) Application number: **24219080.9**

(22) Date of filing: **11.12.2024**

(51) International Patent Classification (IPC):
***C07D 213/75*** *(2006.01)* ***C07C 275/28*** *(2006.01)*
***C07F 15/04*** *(2006.01)* ***C07F 15/06*** *(2006.01)*
***C22B 3/00*** *(2006.01)* ***C22B 23/00*** *(2006.01)*

(52) Cooperative Patent Classification (CPC):
**C07D 213/75; C07C 275/28; C07F 15/045; C07F 15/065; C22B 3/1616; C22B 7/006; C22B 23/0407; C22B 23/0476; C22B 26/12; C22B 47/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**



(71) Applicant: **Technische Universität Dresden**
**01069 Dresden (DE)**

(72) Inventors:
- **WEIGAND, Jan**
  **01309 Dresden (DE)**
- **WENZEL, Marco**
  **01157 Dresden (DE)**
- **LI, Sai**
  **01069 Dresden (DE)**
- **SCHWEDTMANN, Kevin**
  **01159 Dresden (DE)**

(74) Representative: **Maiwald GmbH**
**Elisenhof**
**Elisenstraße 3**
**80335 München (DE)**


(54) **OLIGO-POLYETHER-LIGANDS FOR EXTRACTION OF METAL IONS**


(57) The present invention is directed to new compounds that can be applied in a process of ligand-assisted solid-liquid extraction of metal ions from a mixture comprising a metal salt, to metal complexes of such compounds, to solutions of such metal complexes in aliphatic alcohols, and to the process as such. The new compounds according to the present invention are urea derivatives containing an aromatic or heteroaromatic ring attached to one nitrogen atom of urea and containing at least one polyether group attached to the other nitrogen atom of urea. The new compounds can be dissolved in typical solvents like ethanol and 1-butanol. Depending on the solvent used, these compounds enable a selective extraction of Ni(II) or Co(II) from the respective sulfate salts. Using ethanol as a solvent, the new compounds are usable for extraction of Ni(II) from a mixture of $Li_2SO_4$, $CoSO_4$, $NiSO_4$ and $MnSO_4$ into solution. Using 1-butanol as a solvent, the new compounds are usable for extraction of Co(II) from a mixture of $Li_2SO_4$, $CoSO_4$, $NiSO_4$ and $MnSO_4$ into solution.


EP 4 759 803 A1

## Description

The present invention is directed to oligo-polyether-ligands for extraction of metal ions, i.e., to the compounds usable as ligands as such, that can be applied in a process of ligand-assisted solid-liquid extraction of metal ions from a mixture comprising one or more metal salts, to metal complexes of such compounds, to solutions of such metal complexes in aliphatic alcohols, and to the process of ligand-assisted solid-liquid extraction of metal ions from a mixture comprising one or more metal salts as such.

Intensive work is being carried out on processes for the recovery of metals to reduce $CO_2$ emissions and ensure the supply of critical raw materials to industry. Particularly when using secondary sources such as used materials or waste streams in the context of urban mining, complex material mixtures are present that require innovative processing strategies allowing a) to work economically, and b) to offer solutions for the utilisation of future waste streams.

This aim applies particularly to lithium-ion batteries (LIBs). Spent LIBs are of particular interest for metal recovery due to the increasing use of LIBs to reduce the $CO_2$ emissions, especially in the transport sector, and their high content of the valuable metals nickel and cobalt. In this regard, in the processing of waste streams originating from spent LIBs, the separation of nickel and cobalt from other constituents of spent LIBs is of high interest, as well as the separation of nickel and cobalt from each other.

In this regard, legal requirements require increased recovery rates and the use of recyclates, which secure a long-term sales market for recovered metals or metal salts.

For the processing of secondary raw materials, a two-stage or multi-stage hydrometallurgical process is often used to first dissolve the starting material with suitable reagents (acid or base), followed by the separation of the target metal ions and accompanying elements. The aim is commonly to achieve complete or nearly complete dissolution in order to enable the recovery of the valuable metals as completely as possible. This often requires harsh conditions such as high acid concentrations and/or elevated temperatures. Before downstream processes, such as liquid-liquid extraction or precipitation, can be applied for metal separation operation, it is necessary to regulate process parameters like the acid/base concentration. In particular, the adjustment to a defined pH value requires the addition of auxiliary materials that lead to waste streams. Furthermore, a complete dissolution of the starting material leads to large volume and mass flows in the separation process. The use of reagents and the handling of these volume and mass flows reduce the economic efficiency of the processes, especially with lower quality starting materials.

The separation of nickel and cobalt from a mixture and the separation of the two elements in the form of their ions is currently mainly carried out by liquid-liquid extraction. Depending on the source material, this process is often coupled with various specific procedures to process the respective source material. For example, Co(II) and Ni(II) can be separated from hydrochloric acid solution in the presence of Fe(III) using a trialkylamine such as Alamine 336 in dodecanol and paraffin as the organic phase (Rydberg, J. (Ed.). (2004). Solvent Extraction Principles and Practice, Revised and Expanded (2nd ed.). CRC Press. https://doi.org/10.1201/9780203021460). As the concentration of chloride ions increases, Fe(III) and Co(II) are gradually extracted as their anionic chlorido complexes.

In addition, various classes of substances such as carboxylic acids, hydroxyoximes, hydroxyquinolines, ß-diketones, organophosphoric acids, organophosphanes, organothiophosphates, sulfonic acids, and various N-donor systems as well as their mixtures were reported and experimentally investigated for the separation of the two ions Co(II) and Ni(II) and for the separation of accompanying elements (P. A. Tasker, E. D. Doidge, in Compr. Coord. Chem. IIII (ed.: E.C. Constable, G. Parkin, L. Que Jr), Elsevier, Oxford, 2021, 494-557; C. Y. Cheng, K. R. Barnard, W. Zhang, D. J. Robinson, Solvent Extr. Ion Exch. 2011, 29, 719-754).

Furthermore, the skilled person knows processes in which the valuable metals are separated after acidic leaching by fractional precipitation using precipitation reagents and a variation of the pH value or processes employing other separation methods using specific solvents such as Deep Eutectic Solvents (DES) or Ionic Liquids (IL). The latter, for example, allow the targeted dissolution of individual components, but have a number of other disadvantages.

Furthermore, many of the solvents used are very expensive.

The object of the present invention is to provide an efficient and resource-saving alternative to existing processes for separating Ni(II) and/or Co(II) from mixtures comprising one or more metal salts and for separating Ni(II) and/or Co(II) from each other.

The problem is solved according to the present invention by providing new compounds (ligands) that can be applied in a process of ligand-assisted solid-liquid extraction of metal ions from a mixture comprising one or more metal salts.

The problem is also solved according to the present invention by providing a process of ligand-assisted solid-liquid extraction of metal ions from a mixture comprising one or more metal salts, wherein metal ions can be selectively extracted.

The present invention is according to a first aspect directed to a compound of the following Formula (I),

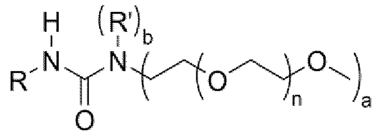

(I),

wherein R is according to the following Formula (II),

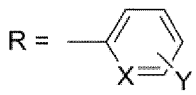

(II),

wherein X is either CH or N,
wherein Y is selected from the group consisting of $CH_3$, $CF_3$, $C_4H_9$, a linear or branched aliphatic alkoxy group comprising from 1 to 12 carbon atoms, $NO_2$, a halogen atom, and an aromatic or heteroaromatic ring comprising 4 to 6 carbon atoms and 0 to 2 nitrogen atoms, wherein the number of group Y is 0, 1, 2, 3, 4, or, in case X is CH, 5, wherein, in case of more than one group Y, the groups Y may be the same or different, wherein R' is either a linear, branched or cyclic aliphatic group comprising 3 to 8 carbon atoms, or is an aromatic group comprising 5 to 14 carbon atoms and 0 to 2 nitrogen atoms in 1, 2, or 3 rings,
wherein n is 0, 1, 2, 3, or 4,
wherein a is 1 or 2,
wherein b is 0 or 1, and
wherein a + b = 2.
X is preferably N.
Y is preferably selected from the group consisting of $CH_3$, $CF_3$, $C_4H_9$, a linear or branched aliphatic alkoxy group comprising from 1 to 12 carbon atoms, $NO_2$, and a halogen atom, more preferably selected from the group consisting of $CH_3$, $CF_3$, $C_4H_9$, a linear or branched aliphatic alkoxy group comprising from 1 to 12 carbon atoms, and a halogen atom, still more preferably selected from the group consisting of $CH_3$, $CF_3$, a linear or branched aliphatic alkoxy group comprising from 1 to 12 carbon atoms, and a halogen atom.

The linear or branched aliphatic alkoxy group comprising from 1 to 12 carbon atoms preferably does not comprise any heteroatoms, i.e., consists of carbon and hydrogen atoms. The linear or branched aliphatic alkoxy group comprises preferably from 1 to 10 carbon atoms, more preferably from 1 to 6 carbon atoms, most preferably from 1 to 4 carbon atoms. The aliphatic alkoxy group is preferably a methoxy or an ethoxy group.

The halogen atom is selected from the group consisting of F, Cl, Br, and I, preferably is selected from the group consisting of F, Cl, and Br, more preferably is selected from the group consisting of F and Cl, and most preferably is F.

The aromatic or heteroaromatic ring comprising 4 to 6 carbon atoms and 0 to 2 nitrogen atoms is preferably connected with a single bond. It preferably does not comprise any further heteroatoms apart from nitrogen. More preferably, it does not comprise any heteroatom, i.e., consists of carbon and hydrogen atoms, and the number of the carbon atoms is 6. The number of group Y is preferably 0, 1, 2, 3, or 4, more preferably 0, 1, 2, or 3, still more preferably 0, 1, or 2, yet still more preferably 0 or 1, and most preferably 0.

Preferably, R' does not comprise any heteroatoms apart from nitrogen.

R' is preferably a linear, branched or cyclic aliphatic group comprising 3 to 8 carbon atoms. More preferably, the linear, branched or cyclic aliphatic group comprising 3 to 8 carbon atoms does not comprise any heteroatoms, i.e., consists of carbon and hydrogen atoms. The amount of carbon atoms is preferably from 3 to 7, more preferably from 3 to 6, still more preferably from 4 to 6, yet still more preferably 5 or 6, and most preferably 6.

Suitable aliphatic groups are methyl, ethyl, propyl, iso-propyl, cyclopropyl, n-butyl, tert-butyl, cyclobutyl, n-pentyl, iso-pentyl (3-methylbutyl), cyclopentyl, n-hexyl, isohexyl, and cyclohexyl.

Particularly preferred are cyclopentyl and cyclohexyl, with cyclohexyl being most preferred.

In case R' is an aromatic group, the amount of carbon atoms is preferably from 5 to 14 and the amount of nitrogen atoms is 0 in 1, 2, or 3 rings, more preferably the amount of carbon atoms is from 5 to 12 and the amount of nitrogen atoms is 0 in 1 or 2 rings, still more preferably the amount of carbon atoms is from 5 to 6 and the amount of nitrogen atoms is 0 in 1 ring, and most preferably the amount of carbon atoms is 6 and the amount of nitrogen atoms is 0 in 1 ring.

Preferably, n is 0, 1, 2, or 3, more preferably 0, 1, or 2, still more preferably 0 or 1, and most preferably 1.

According to one preferred embodiment of the first aspect of the present invention, the compound of Formula (I) is defined as follows:

X is either CH or N,
Y is selected from the group consisting of $CH_3$, $CF_3$, $C_4H_9$, a linear or branched aliphatic alkoxy group comprising from 1 to 12 carbon atoms, $NO_2$, and a halogen atom,
the number of group Y is 0, 1, 2, or 3,
wherein, in case of more than one group Y, the groups Y are the same,
R' is a linear, branched or cyclic aliphatic group comprising 3 to 8 carbon atoms,
n is 0, 1, or 2,
a is 1 or 2,
b is 0 or 1, and

$$a + b = 2.$$

According to another preferred embodiment of the first aspect of the present invention, the compound of Formula (I) is defined as follows:

X is either CH or N,
Y is selected from the group consisting of $CH_3$, $CF_3$, $C_4H_9$, a linear or branched aliphatic alkoxy group comprising from 1 to 12 carbon atoms, $NO_2$, and a halogen atom,
the number of group Y is 0 or 1,
R' is a linear, branched or cyclic aliphatic group comprising 3 to 8 carbon atoms,
n is 0, 1 or 2,
a is 1 or 2,
b is 0 or 1, and

$$a + b = 2.$$

According to another preferred embodiment of the first aspect of the present invention, the compound of Formula (I) is defined as follows:

X is either CH or N,
the number of group Y is 0,
R' is a linear, branched or cyclic aliphatic group comprising 3 to 8 carbon atoms,
n is 0, 1 or 2,
a is 1 or 2,
b is 0 or 1, and

$$a + b = 2.$$

According to another preferred embodiment of the first aspect of the present invention, the compound of Formula (I) is defined as follows:

X is either CH or N,
the number of group Y is 0,
R' is a linear, branched or cyclic aliphatic group comprising 3 to 6 carbon atoms,
n is 0 or 1,
a is 1 or 2,
b is 0 or 1, and

$$a + b = 2.$$

According to another preferred embodiment of the first aspect of the present invention, the compound of Formula (I) is defined as follows:

X is either CH or N,
the number of group Y is 0,
R' is a linear, branched or cyclic aliphatic group comprising 3 to 6 carbon atoms,
n is 1,
a is 1 or 2,

b is 0 or 1, and

$$a + b = 2.$$

Referring to the general definition of the first aspect of the present invention and to its preferred embodiments indicated above, the first aspect of the present invention also covers embodiments, wherein the following combinations 1), 2), and 3) of the definition of Formula (I) are not included into the scope of the first aspect of the present invention but the remainder of the definitions of Formula (I) remain as defined above:

1) X is CH, and Y is $NO_2$, and the number of group Y is 1, and R' is phenyl, and n is 1, and a is 1, and b is 1;
2) R is phenyl (X being CH and the number of group Y being 0), and n is 0, and a is 2, and b is 0;
3) R is 2-pyridyl (X being N and the number of group Y being 0), and n is 0, and a is 2, and b is 0.

The present invention is according to a second aspect directed to a complex of a compound of Formula (I) according to the first aspect of the present invention, including all its preferred embodiments, with a metal ion.

According to a preferred embodiment of the second aspect of the present invention, the metal ion is selected from the group consisting of ions of Co and Ni.

Furthermore, it is preferred that the ions of Co and Ni are present in the complex as Co(II) and Ni(II).

The present invention is according to a third aspect directed to a solution of a complex according to the second aspect of the present invention, including all its preferred embodiments, in a solvent comprising an alcohol of the formula $R^A$-OH, wherein $R^A$ is a linear or branched alkyl residue having from 1 to 6 carbon atoms, preferably from 1 to 5 carbon atoms, more preferably from 2 to 4 carbon atoms.

Suitable alcohols of the formula $R^A$-OH are methanol, ethanol, 1-propanol, 2-propanol, 1-butanol, 2-butanol, tert-butanol, e.g., 1-pentanol, 2-pentanol, 3-pentanol, 1-isopentanol (3-methyl-1-butanol), 2-metyl-2-butanol, 3-metyl-2-butanol, 1-hexanol, 2-hexanol, 3-hexanol, 2,3-dimethyl-2-butanol, 3,3-dimethyl-1-butanol, 2-methyl-1-pentanol, 3-methyl-1-pentanol, 4-methyl-1-pentanol, 2-methyl-2-pentanol, 3-methyl-2-pentanol, 4-methyl-2-pentanol, 2-methyl-3-pentanol, 3-methyl-3-pentanol, cyclohexanol, 1-ethyl-1-cyclobutanol, and 2-ethyl-1-cyclobutanol.

Preferred alcohols of the formula $R^A$-OH are methanol, ethanol, 1-propanol, 2-propanol, 1-butanol, 2-butanol, tert-butanol, 1-pentanol, 2-pentanol, 3-pentanol, and 1-isopentanol (3-methyl-1-butanol).

Particular preferred alcohols of the formula $R^A$-OH are ethanol, 1-propanol, 2-propanol, 1-butanol, and 2-butanol.

The most preferred alcohols of the formula $R^A$-OH are ethanol and 1-butanol.

Preferably, in the solution according to the third aspect of the present invention, the alcohol of the formula $R^A$-OH makes up at least 50 wt%, more preferably at least 75 wt%, still more preferably at least 90 wt%, yet more preferably at least 95 wt% or 99 wt% of the solvent, like 95 to 100 wt% or 99 to 100 wt% of the solvent. According to a particularly preferred embodiment, the solvent consists of the alcohol of the formula $R^A$-OH. This latter definition allows the presence of unavoidable trace amounts of water.

The present invention is according to a fourth aspect directed to a process of ligand-assisted solid-liquid extraction of metal ions from a mixture comprising a metal salt, the process comprising the following steps in the given order:

a) providing a solid mixture comprising a metal salt,
b) providing a compound of Formula (I) as defined in the first aspect of the present invention, including all its preferred embodiments,
c) dissolving the compound of Formula (I) in a solvent comprising an alcohol of the formula $R^A$-OH and obtaining a solution of the compound of Formula (I), wherein the alcohol of the formula $R^A$-OH is as defined in connection with the third aspect of the present invention, including all its preferred embodiments,
d) putting the solid mixture comprising a metal salt of step a) and the solution of step c) together in a container,
e) setting entire container or content of the container in motion, such that all parts of the solid mixture comprising a metal salt of step a) come into contact with the solution of step c),
f) stopping moving the container or the content of the container and allowing separation of a solid phase and a liquid phase from each other in the container,
g) removing the liquid phase from the container.

The liquid phase obtained from step g) contains the extracted metal ions.

The solid mixture comprising a metal salt of step a) is a mixture of solids, which mixture comprises one or more metal salts.

The container in step d) can be any conventional container, preferably a sealable container, preferably a

container which fits into a shaker and into a centrifuge, like a microcentrifuge tube.

The movement of the container or content of the container in step e) can be performed in any conventional manner using conventional devices like a shaker, e.g., an overhead shaker or a horizontal shaker, or by stirring.

The movement of the container or content of the container in step e) is preferable performed for 8 to 16 hours, e.g., overnight.

The separation of a solid phase and a liquid phase from each other in the container in step f) can be performed in any conventional manner, e.g., by upright storage of the container for a suitable time span, like 1 to 30 minutes. The separation of a solid phase and a liquid phase from each other in the container in step f) can be supported by centrifugation of the container.

The removal of the liquid phase from the container in step g) can be performed in any conventional manner, e.g., by using a pipette.

The liquid phase removed from the container in step g) may be, preferably is, filtrated in a conventional manner to remove residual solids.

As indicated above, the liquid phase obtained from step g) contains the extracted metal ions. Accordingly, the process of the fourth aspect of the present invention enables dissolving metal ions from a solid mixture comprising a metal salt, i.e., the solid mixture comprising one metal salt or different metal salts.

Preferably, the solid mixture comprising a metal salt of step a) comprises a salt of Co(II), a salt of Ni(II), or mixtures thereof.

The phrase "a salt of Co(II)" covers both merely one type of Co(II) salt or different Co(II) salts. The phrase "a salt of Ni(II)" covers both merely one type of Ni(II) salt or different Ni(II) salts.

The process of the fourth aspect of the present invention enables dissolving Co(II) and Ni(II) from a solid mixture comprising a salt of Co(II), a salt of Ni(II), or mixtures thereof, which solid mixture may comprise additional metal salts.

More preferably, the solid mixture comprising a metal salt of step a) comprises a salt of Li(I), a salt of Mn(II), a salt of Co(II), and a salt of Ni(II).

The phrase "a salt of Mn(II)" covers both merely one type of Mn(II) salt or different Mn(II) salts. The phrase "a salt of Li(I)" covers both merely one type of Li(I) salt or different Li(I) salts.

The process of the fourth aspect of the present invention enables dissolving Co(II) and Ni(II) from a solid mixture comprising a salt of Li(I), a salt of Mn(II), a salt of Co(II), and a salt of Ni(II), which solid mixture may comprise additional metal salts.

Still more preferably, the solid mixture comprising a metal salt of step a) comprises oxoanion salts of Li(I), Mn(II), Co(II), and Ni(II).

Still more preferably, the solid mixture comprising a metal salt of step a) comprises sulfate salts of Li(I), Mn(II), Co(II), and Ni(II).

Yet more preferably, the solid mixture comprising a metal salt of step a) comprises $Li_2SO_4$, $MnSO_4 \cdot H_2O$, $CoSO_4 \cdot 7H_2O$, and $NiSO_4 \cdot 6H_2O$.

Preferably, the sum of the salts of Li(I), Mn(II), Co(II), and Ni(II) makes up at least 50 wt%, more preferably at least 75 wt%, still more preferably at least 90 wt%, yet more preferably at least 95 wt% or at least 99 wt%, like 95 to 100 wt% or 99 to 100 wt%, most preferably consists, of the sum of all metal salts in the solid mixture comprising a metal salt of step a).

Preferably, in the solvent of step c) the alcohol of the formula $R^A$-OH makes up at least 50 wt%, more preferably at least 75 wt%, still more preferably at least 90 wt%, yet more preferably at least 95 wt% or at least 99 wt% of the solvent, like 95 to 100 wt% or 99 to 100 wt% of the solvent. According to a particularly preferred embodiment, the solvent consists of the alcohol of the formula $R^A$-OH. This latter definition allows the presence of unavoidable trace amounts of water.

The most preferred alcohols of the formula $R^A$-OH are ethanol and 1-butanol.

The process according to the fourth aspect of the present invention as described above can be performed more than once without providing a fresh solid mixture comprising a metal salt. Instead, after performing the process once, the solid phase of step f) may remain in the container and may optionally be combined with residual solids obtained from filtration of the liquid phase removed from the container in step g). Some residual liquid phase in the solid phase does not hurt. This solid phase can be used as solid mixture comprising a metal salt of step a) for a subsequent run of the process according to the fourth aspect of the present invention as described above. This procedure can be repeated, e.g., until no further metal ions are extracted from the solid mixture.

For a given subsequent run of the process according to the fourth aspect of the present invention, the solvent applied can be the same as in the previous run or the solvent can be different.

Using the same solvent in a subsequent run of the process as in the previous run of the process, selective extraction of a certain metal ion can be continued, and the overall quantity of the certain metal ion, which is extracted from the original solid mixture comprising a metal salt of step a), can be enhanced.

Accordingly, a solid mixture comprising a metal salt of step a) of any subsequent run of the process may comprise a metal salt of a certain metal ion in a smaller amount or may not comprise a metal salt of a certain metal ion anymore following extraction thereof.

Using a different solvent, selective extraction of another metal ion can be performed after selective extraction of said certain metal ion.

According to a particular preferred embodiment of the fourth aspect of the present invention, the solid mixture comprising a metal salt of step a) comprises sulfate salts of Li(I), Mn(II), Co(II), and Ni(II), the process is performed twice, i.e., at least twice, wherein in a subsequent run of the process the solid mixture comprising a metal salt of step a) is obtained from the solid phase referred to in step f) of the process of the previous run of the process, wherein in the previous run of the process the solvent is solvent A, which is different from solvent B, which is applied in a subsequent run of the process, solvent A comprises an alcohol A of the formula $R^A$-OH, which preferably makes up 99 to 100 wt% of the solvent A, and solvent B comprises an alcohol B of the formula $R^A$-OH, which preferably makes up 99 to 100 wt% of the solvent B, wherein still more preferably alcohol A and alcohol B are different and are selected from the group consisting of ethanol and 1-butanol, wherein most preferably alcohol A is ethanol and alcohol B is 1-butanol.

This particular preferred embodiment of the present invention allows selective and subsequent solid-liquid extraction of Co(II) and Ni(II) from a mixture comprising sulfate salts of Li(I), Mn(II), Co(II), and Ni(II), which mixtures are typical in lithium-ion batteries.

As can be taken from the detailed examples below, using ethanol as solvent allows selective extraction of Ni(II) with high quantities. Furthermore, using 1-butanol as solvent, selective extraction of Co(II) and some Ni(II) is possible.

Hence, in case separation of Ni(II) and Co(II) is desired, it is preferred to run the process (once or in more cycles) with ethanol as solvent followed by running the process (once or in more cycles) with 1-butanol s solvent.

The present invention is according to a fifth aspect directed to the use of a compound of Formula (I) according to the first aspect of the present invention, including all its preferred embodiments, for extracting metal ions from a solid mixture comprising a metal salt, wherein the metal ions are defined as in the second aspect of the present invention, including all its preferred embodiments, and wherein the solid mixture comprising a metal salt and the metal salts are defined as in the fourth aspect of the present invention, including all its preferred embodiments.

## Experimental Part

### Chemicals

If not stated differently, all manipulations were performed with HPLC grade, analytical grade or technical grade reagents and solvents, which were used without further purification. The used starting materials, auxiliaries and solvents, triethylamine (EtsN, 99.5%), ethyl acetate (EtOAc), iso-hexane, chloroform ($CHCl_3$), dichloromethane (DCM, $CH_2Cl_2$), acetonitrile ($CH_3CN$), tetrahydrofuran (THF), toluene, diethylether ($Et_2O$), acetone, methanol (MeOH), ethanol (EtOH), 1-butanol, $Cs_2CO_3$ (99%), Pd/C catalyst (10 wt.% loading), phenyl isocyanate (98%), picolinic acid (99%), diphenylphosphoryl azide (DPPA, 86%), ammonium formate ($NH_4(HCOO)$, 99%), $NaBH_4$ (98%), NaI (99%), NaOH (99%), $NaHCO_3$ (99%), $K_2CO_3$ (99.9%), $Li_2SO_4$ (99%), $MnSO_4 \cdot H_2O$ (99%), $CoSO_4 \cdot 7H_2O$ (99%), $NiSO_4 \cdot 6H_2O$ (99%), $Na_2SO_4$, $MgSO_4$, HNOs (69%) were purchased from *SIGMA-ALDRICH*, *FLUKA*, *MERCK*, *VWR*, *TCI*, *ABCR CHEMICALS*, *ACROS*, *CARL ROTH* and *WAKO.* Ultrapure water (18.2 MΩ·cm, arium pro, Sartorius) was used for the preparation of diluted HNOs. Manipulations under inert atmosphere were performed in a Glovebox MB Unilab or using *Schlenk* techniques under an atmosphere of purified nitrogen or argon. All glassware was oven-dried at 160 °C prior to use. Dry, oxygen-free solvents were employed ($CH_2Cl_2$, $CH_3CN$, and EtsN were distilled from $CaH_2$ and n-hexane, $Et_2O$ and toluene were distilled from potassium). Anhydrous $CDCl_3$, $CD_2Cl_2$, $CD_3CN$, $CD_3OD$ and DMSO-$d_6$ were purchased from Sigma-Aldrich, Deutero or Eurisotop and dried over molecular sieve. All distilled and deuterated solvents were stored over molecular sieves (4 Å: $CH_2Cl_2$, $CD_2Cl_2$, $CDCl_3$, $Et_2O$, 1,4-dioxane, n-pentane, n-hexane, toluene, toluene-$d_8$; 3 Å: $CH_3CN$, $CD_3CN$).

### Methods and Instruments

NMR spectra were measured on a Bruker AVANCE III HD Nanobay 400 MHz UltraSield ($^1H$ (400.13 MHz), $^{13}C$ (100.61 MHz), $^7Li$ (155.51 MHz)) or on a Bruker AVANCE III HDX, 500 MHz Ascend ($^1H$ (500.13 MHz), $^{13}C$ (125.75 MHz), $^7Li$ (194.37 MHz)). All $^{13}C$ NMR spectra were exclusively recorded with composite pulse decoupling. Reported numbers assigning atoms in the $^{13}C$ spectra were indirectly deduced from the cross-peaks in 2D correlation experiments (HMBC, HSQC). Chemical shifts were referenced to the respective solvent to $\delta$ = 7.26 ppm ($^1H$), 77.16 ppm ($^{13}C$) for $CDCl_3$, $\delta$ = 5.32 ppm ($^1H$), 53.84 ppm ($^{13}C$) for $CD_2Cl_2$, $\delta$ = 1.94 ppm ($^1H$), 118.26 ppm ($^{13}C$) for $CD_3CN$, $\delta$ = 3.31 ppm ($^1H$), 49.00 ppm ($^{13}C$) for $CD_3OD$). Chemical shifts ($\delta$) are reported in ppm. Coupling constants (*J*) are reported in Hz.

Infrared (IR) and Raman spectra were recorded at ambient temperature using a Bruker

Vertex 70 instrument equipped with a RAM II module (Nd-YAG laser, 1064 nm). The Raman intensities are reported in percent relative to the most intense peak and are given in parenthesis. An ATR (attenuated total reflectance)

unit (diamond) was used for recording IR spectra. The intensities are reported relative to the most intense peak and are given in parenthesis using the following abbreviations: vw = very weak, w = weak, m = medium, s = strong, vs = very strong.

Elemental analyses were performed on a Vario MICRO cube Elemental Analyzer by Elementar Analysatorsysteme GmbH in CHNS modus.

For the mass spectrometry (MS) experiments, a waters ACQUITY UPLC H-Class system in combination with an ACQUITY TQ Detector V4.1 SCN849 SCN896 was used. MassLynx V4.1 SCN849 SCN896 served as the evaluation software. The required ionization was provided by the electrospray method (ESI). The diluent for the samples was an acetonitrile water mixture containing both solvents in a 70:30 (acetonitrile:water) ratio and additionally 0.1% of formic acid.

The concentration of metals in solution were determined by inductively coupled plasma-optical emission spectrometry (ICP-OES, OPTIMA 2000DV, PerkinElmer and 5900 SVDV, Agilent). The pH values were measured by a pH-meter (type 765 Calimatic, Knick, Germany) with an InlabMicro-electrode (Mettler Toledo).

**Solid-liquid Extraction Studies**

Solid-liquid extraction (SLE) experiments are performed in microcentrifuge tubes. A solution (1 mL) of ligand in a suitable alcohol like ethanol or 1-butanol (40 mM) is layered over a solid mixture of $Li_2SO_4$ (22.0 mg, 0.2 mmol), $MnSO_4 \cdot H_2O$ (33.8 mg, 0.2 mmol), $CoSO_4 \cdot 7H_2O$ (56.2 mg, 0.2 mmol), $NiSO_4 \cdot 6H_2O$ (52.6 mg, 0.2 mmol) (5 times molar excess, $2 \times 10^{-4}$ M) and agitated overnight in an overhead shaker. The mixture is centrifuged, and the collected liquid is filtered through 200 nm filter. 0.1 mL filtrate is directly diluted to 6 mL with 0.1 M $HNO_3$, and the metal ion concentration is determined by ICP-OES analysis. Control experiments are carried out in parallel using the solvent alone. All extraction experiments are carried out in duplicate, i.e., each presented data point depicted in the results is the average of two individual experiments.

The extraction power of the ligand is quantified by the metal loading, which is calculated by the following equation:

Metal loading in percent = [(determined metal concentration - average concentration blank) × dilution/(molar mass (metal) × c (ligand))] × 100

The determined metal concentration is the metal concentration of the sample (in mol%) as determined by ICP-OES analysis of the metal for which the metal loading is calculated.

The average concentration blank is the metal concentration of the blank (control experiment, solvent only) (in mol%) as determined by ICP-OES analysis of the metal for which the metal loading is calculated.

The dilution is the dilution, which is obtained by adding HNOs to the sample.

The molar mass (metal) is the molar mass of the metal for which the metal loading is calculated.

The concentration c(ligand) is the concentration of the ligand present in the organic phase in the extraction experiment.

**The Ligands**

The following formula shows the urea derivatives, which are used as ligands in the following extraction experiments.

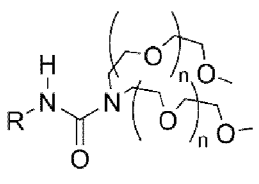

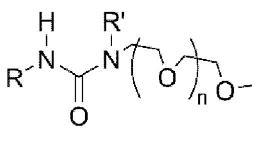

**1**: R = $C_6H_5$, n = 1
**2**: R = $C_6H_5$, n = 0
**3**: R = 2-Py, n = 1
**4**: R = 2-Py, n = 0

**5**: R = $C_6H_5$, R' = $C_6H_{11}$, n = 1
**6**: R = 2-Py, R' = $C_6H_{11}$, n = 1

**Extraction and Reference Examples**

The following extraction examples are performed as described above for the solid-liquid extraction studies using ligands as indicated herein above.

For each of the extraction examples also a reference example is performed, which differs from the corresponding

extraction example merely insofar as no ligand is applied, i.e., using the solvent alone.

The metal loading is determined for each pair of experiments and is indicated in one diagram each in the figures disclosed herein. The result of the reference examples is indicated as "blank" in the figures disclosed herein.

**Extraction Example 1**

Ligand: 1,1-bis(2-(2-methoxyethoxy)ethyl)-3-(pyridin-2-yl)urea **(3).**
Solvent: ethanol.

Three independent identical experiments of Extraction Example 1 are carried out.

Figure 1 shows the metal loadings of three independent identical experiments of Extraction Example 1, including the corresponding reference example.

The determined nickel loading of the ligand is higher than 98% while the loading of the other metal ions is below 2%.

The comparison of Extraction Example 1 with the corresponding reference example shows that selective extraction of Ni(II) from the metal salt mixture is possible and that apparently very low amounts of Ni(II) and Li(I) are soluble in the solvent ethanol.

Figure 2 shows the microcentrifuge tubes containing the solid-liquid mixtures of Extraction Example 1 (left) and of the corresponding reference example (right).

The green colour of the solution of Extraction Example 1 is indicative of the nickel complex of the ligand in solution, while the solution of the corresponding reference example is not coloured.

Additional evidence for the selective separation of Ni(II) by a ligand-assisted extraction is provided by the results of electrospray ionization mass spectrometry (ESI-MS) analysis of the solution.

The results are shown in Figure 11.

The signal at m/z = 342.1 is assigned to the free ligand **3** ([**3**+H]$^+$). With positive ionization, a signal is observed at m/z = 739.2 that can be assigned to a species [Ni·**3**·(**3**-H)]$^+$, an adduct of two ligands and one Ni(II). Good agreement is observed between the recorded data and the simulated isotope patterns.

Hence, if ethanol is used as solvent, there is evidence of the extraction of Ni(II) only.

**Extraction Example 2**

Ligand: 1,1-bis(2-(2-methoxyethoxy)ethyl)-3-(pyridin-2-yl)urea **(3).**
Solvent: 1-butanol.

Three independent identical experiments of Extraction Example 2 are carried out.

Figure 3 shows the metal loadings of three independent identical experiments of Extraction Example 2, including the corresponding reference example.

The determined cobalt loading of the ligand is up to 72% while the loading of the other metal ions is low (nickel: up to 7%; lithium: up to 2%).

The comparison of Extraction Example 2 with the corresponding reference example shows that selective extraction of Co(II) from the metal salt mixture is possible, that some minor amount of Ni(II) is extracted as well, and that apparently low amounts of Co(II) and very low amounts of Li(I) are soluble in the solvent 1-butanol, while the solubility of Ni(II) is apparently neglectable.

Figure 4 shows the microcentrifuge tubes containing the solid-liquid mixtures of Extraction Example 2 (left) and of the corresponding reference example (right).

The pink colour of the solution of Extraction Example 2 is indicative of the cobalt complex of the ligand in solution, while the solution of the corresponding reference example is not coloured.

Additional evidence for the selective separation of Co(II) by a ligand-assisted extraction is provided by the results of electrospray ionization mass spectrometry (ESI-MS) analysis of the solution.

The results are shown in Figure 12.

The signal at m/z = 342.1 is assigned to the free ligand **3** ([**3**+H]$^+$). With positive ionization, a signal is observed at m/z = 740.3 that can be assigned to a species [Co·**3**·(**3**-H)]$^+$, an adduct of two ligands and one Co(II). Good agreement is

observed between the recorded data and the simulated isotope patterns.

Hence, if 1-butanol is used as solvent, there is evidence of the extraction of Co(II) only.

**Extraction Example 3**

| | |
|---|---|
| Ligand: | 1,1-bis(2-(2-methoxyethoxy)ethyl)-3-phenylurea **(1).** |
| Solvent: | ethanol. |

Merely one experiment of Extraction Example 3 is carried out.

Figure 5 shows the metal loading of the experiment of Extraction Example 3, including the corresponding reference example.

The determined nickel loading of the ligand is around 36% while the loading of the other metal ions is not detectable with the exception of Li(I) (below 2%).

The comparison of Extraction Example 3 with the corresponding reference example shows that selective extraction of Ni(II) from the metal salt mixture is possible and that apparently very low amounts of Ni(II) and Li(I) are soluble in the solvent ethanol. The result is insofar qualitatively the same as in Extraction Example 1.

**Extraction Example 4**

| | |
|---|---|
| Ligand: | 1,1-bis(2-methoxyethyl)-3-(pyridin-2-yl)urea **(4).** |
| Solvent: | ethanol. |

Merely one experiment of Extraction Example 4 is carried out.

Figure 6 shows the metal loading of the experiment of Extraction Example 4, including the corresponding reference example.

The determined nickel loading of the ligand is around 98% while the loading of the other metal ions is not detectable with the exception of Li(I) (below 2%).

The comparison of Extraction Example 4 with the corresponding reference example shows that selective extraction of Ni(II) from the metal salt mixture is possible and that apparently very low amounts of Ni(II) and Li(I) are soluble in the solvent ethanol. The result is insofar qualitatively the same as in Extraction Example 1.

**Extraction Example 5**

| | |
|---|---|
| Ligand: | 1-cyclohexyl-1-(2-(2-methoxyethoxy)ethyl)-3-(pyridin-2-yl)urea **(6).** |
| Solvent: | ethanol. |

Merely one experiment of Extraction Example 5 is carried out.

Figure 7 shows the metal loading of the experiment of Extraction Example 5, including the corresponding reference example.

The determined nickel loading of the ligand is around 91% while the loading of the other metal ions is not detectable with the exception of Li(I) (below 2%).

The comparison of Extraction Example 4 with the corresponding reference example shows that selective extraction of Ni(II) from the metal salt mixture is possible and that apparently very low amounts of Ni(II) and Li(I) are soluble in the solvent ethanol. The result is insofar qualitatively the same as in Extraction Example 1.

**Extraction Example 6**

Ligand: 1,1-bis(2-(2-methoxyethoxy)ethyl)-3-phenylurea **(1).**
Solvent: 1-butanol.

Merely one experiment of Extraction Example 6 is carried out.
Figure 8 shows the metal loading of the experiment of Extraction Example 6, including the corresponding reference example.
The determined cobalt loading of the ligand is below 5% while the loading of the other metal ions is not detectable.
The comparison of Extraction Example 6 with the corresponding reference example shows that selective extraction of Co(II) from the metal salt mixture is possible and that apparently very low amounts of Li(I) are soluble in the solvent 1-butanol, while the solubility of Ni(II) is apparently neglectable. The result is insofar qualitatively the same as in Extraction Example 2.

**Extraction Example 7**

Ligand: 1,1-bis(2-methoxyethyl)-3-(pyridin-2-yl)urea **(4).**
Solvent: 1-butanol.

Merely one experiment of Extraction Example 7 is carried out.
Figure 9 shows the metal loading of the experiment of Extraction Example 7, including the corresponding reference example.
The determined cobalt loading of the ligand is around 51% while the loading of the other metal ions is low (nickel: 6%; lithium: below 1%).
The comparison of Extraction Example 7 with the corresponding reference example shows that selective extraction of Co(II) from the metal salt mixture is possible, that some minor amount of Ni(II) is extracted as well, and that apparently low amounts of Co(II) and very low amounts of Li(I) are soluble in the solvent 1-butanol, while the solubility of Ni(II) is apparently neglectable. The result is insofar qualitatively the same as in Extraction Example 2.

**Extraction Example 8**

Ligand: 1-cyclohexyl-1-(2-(2-methoxyethoxy)ethyl)-3-(pyridin-2-yl)urea **(6).**
Solvent: 1-butanol.

Merely one experiment of Extraction Example 8 is carried out.
Figure 10 shows the metal loading of the experiment of Extraction Example 8, including the corresponding reference example.
The determined cobalt loading of the ligand is around 20% while the loading of the other metal ions is low (nickel: around 2%; lithium: below 1%).
The comparison of Extraction Example 8 with the corresponding reference example shows that selective extraction of Co(II) from the metal salt mixture is possible, that some minor amount of Ni(II) is extracted as well, and that apparently low amounts of Co(II) and very low amounts of Li(I) are soluble in the solvent 1-butanol, while the solubility of Ni(II) is apparently neglectable. The result is insofar qualitatively the same as in Extraction Example 2.

**Recovery of the Separated Metal Salts and of the Ligand**

After solid-liquid extraction, the samples are filtered for solid/liquid separation. The solvent of the obtained liquid is evaporated to dryness. The crude mixture is treated with dichloromethane to dissolve the ligand, whereas the metal salt ($NiSO_4$ or $CoSO_4$) as well as the corresponding complexes are not dissolved (Fig. 13 a-c).
In addition to the bare eye observation (colourful residue and colourless solution), the proton NMR spectra of both solutions obtained after solid-liquid extraction of Ni(II) and Co(II), respectively, with **3** show the same set of resonances compared to a sample of **3** dissolved in $CD_2Cl_2$ that has not been used in an extraction experiment, indicating that the free receptor is present in solution (Fig. 13 d).

**Synthesis of Ligands**

**Synthesis Example 1**

Synthesis of 1,1-bis(2-(2-methoxyethoxy)ethyl)-3-phenylurea **(1)**

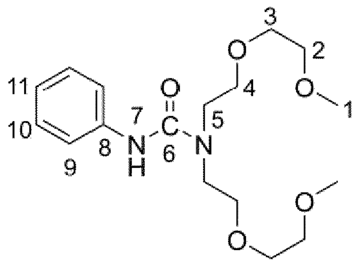

To a solution of bis(2-(2-methoxyethoxy)ethyl)amine (4.00 g, 18.08 mmol, 1.0 eq.) in 50 ml distilled DCM, phenyl isocyanate (2.26 g, 18.98 mmol, 1.05 eq.) is added dropwise at -30 °C. After complete addition, the stirred mixture is warmed up to room temperature and refluxed overnight. After cooling to room temperature, the reaction mixture is filtered, and solvent is removed under reduced pressure. The crude product is purified by column chromatography (silica gel, iso-hexane EtOAc = 6 : 1 ---> 3 : 1) to yield a pale yellow oil.

**Yield:** 3.26 g (53%); **Raman** (100 mW, 298 K, in $cm^{-1}$): 3060 (33), 2982 (23), 2917 (53), 2874 (54), 2821 (56), 1669 (14), 1604 (66), 1503 (5), 1474 (16), 1446 (16), 1311 (19), 1254 (29), 1179 (9), 1157 (10), 1032 (23), 1000 (88), 873 (13), 850 (9), 624 (5), 77 (100); **IR** (ATR, 298 K, in $cm^{-1}$): 3321 (vw), 2874 (w), 1666 (s), 1597 (m), 1543 (s), 1501 (m), 1443 (s), 1397 (w), 1364 (w), 1309 (m), 1251 (m), 1226 (s), 1199 (w), 1097 (vs), 1028 (m), 918 (vw), 873 (vw), 848 (w), 754 (vs), 694 (s), 624 (w), 601 (w), 508 (m), 427 (vw); **$^1$H NMR** ($CDCl_3$, 400 MHz, 300 K, in ppm): $\delta$ = 8.30 (s, 1H, H7), 7.34 (2H, d, $^3J_{HH}$ = 7.64 Hz, H9), 7.19 (2H, t, $^3J_{HH}$ = 7.92 Hz, H10), 6.89 (1H, t, $^3J_{HH}$ = 7.36 Hz, H11), 3.66 (4H, t, $^3J_{HH}$ = 4.76 Hz, H2), 3.60 (4H, m, H3), 3.54 (4H, t, $^3J_{HH}$ = 4.70 Hz, H5), 3.50 (4H, m, H4), 3.32 (6H, s, H1); **$^{13}$C{$^1$H} NMR** ($CDCl_3$, 400 MHz, 300 K, in ppm): $\delta$ = 156.88 (1C, s, C6), 140.20 (1C, s, C8), 128.52 (2C, s, C10), 121.75 (1C, s, C11), 118.86 (2C, s, C9), 71.62 (2C, s, C4), 71.15 (2C, s, C2), 70.48 (2C, s, C3), 58.76 (2C, s, C1), 49.17 (2C, s, C5); **Elemental analysis** for $C_{17}H_{27}N_2O_5$, calculated: C 60.16, H 8.02, N 8.25; found: C 59.65, H 7.97, N 7.97; **ESI-MS** (m/z, [Da/e]): 341.3 $[M+H]^+$ ($ESI^+$).

**Synthesis Example 2**

Synthesis of 1,1-bis(2-methoxyethyl)-3-phenylurea **(2)**

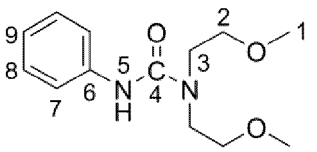

To a solution of bis(2-methoxyethyl)amine (4.00 g, 30.03 mmol, 1.0 eq.) in 50 ml distilled DCM, phenyl isocyanate (4.11 g, 34.54 mmol, 1.15 eq.) is added dropwise at -30 °C. After complete addition, the stirred mixture is warmed up to room temperature and refluxed overnight. After cooling to room temperature, the solvent is removed under reduced pressure. 2 is obtained as a pale yellow oil and is used without further purification.

**Yield:** 6.76 g (89%); **Raman** (100 mW, 298 K, in $cm^{-1}$): 3058 (13), 2983 (10), 2939 (15), 2930 (15), 2902 (13), 2834 (19), 1667 (10), 1603 (44), 1501 (6), 1475 (6), 1444 (10), 1311 (13), 1255 (17), 1177 (8), 1156 (8), 1031 (17), 999 (67), 873 (8), 847 (6), 823 (6), 256 (8), 208 (8), 77 (100); **IR** (ATR, 298 K, in $cm^{-1}$): 3325 (vw), 2986 (vw), 2935 (vw), 2879 (vw), 2829 (vw), 1666 (s), 1597 (m), 1540 (s), 1500 (m), 1460 (m), 1443 (s), 1396 (w), 1363 (w), 1308 (s), 1252 (m), 1231 (m), 1195 (s), 1157 (w), 1110 (vs), 1069 (s), 1012 (s), 965 (w), 873 (m), 844 (w), 800 (w), 751 (vs), 693 (vs), 623 (w), 601 (m), 548 (w), 507 (m), 487 (vw), 415 (vw); **$^1$H NMR** ($CDCl_3$, 400 MHz, 300 K, in ppm): $\delta$ = 8.37 (1H, s, H5), 7.30 (2H, d, $^3J_{HH}$ = 7.56 Hz, H8), 7.23 (2H, t, $^3J_{HH}$ = 7.88 Hz, H7), 6.94 (1H, t, $^3J_{HH}$ = 7.28 Hz, H9), 3.58 (4H, t, $^3J_{HH}$ = 4.52 Hz, H2), 3.53 (4H, t, $^3J_{HH}$ = 4.50 Hz, H3), 3.38 (6H, s, H1); **$^{13}$C{$^1$H} NMR** ($CDCl_3$, 400 MHz, 300 K, in ppm): $\delta$ = 156.86 (1C, s, C4), 140.16 (1C, s, C6),

128.67 (2C, s, C7), 121.93 (1C, s, C9), 118.96 (2C, s, C8), 72.77 (2C, s, C2), 58.94 (2C, s, C1), 49.31 (2C, s, C3); **Elemental analysis** for $C_{13}H_{20}N_2O_3$, calculated: C 61.88, H 7.99, N 11.10; found: C 62.01, H 7.74, N 10.92; **ESI-MS** (m/z, [Da/e]): 253.4 $[M+H]^+$ ($ESI^+$).

## Synthesis Example 3

Synthesis of 1,1-bis(2-(2-methoxyethoxy)ethyl)-3-(pyridin-2-yl)urea **(3)**

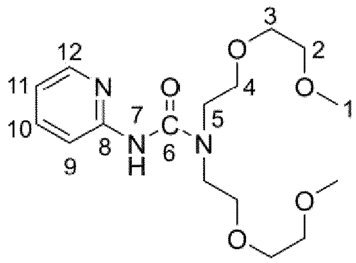

To a suspension of 2-isocyanatopyridine (0.81 g, 6.77 mmol, 1.05 eq.) in 50 ml distilled toluene, bis(2-(2-methoxyethoxy)ethyl)amine (1.43 g, 6.46 mmol, 1.0 eq.) is added dropwise. The suspension is heated to 120 °C and then refluxed overnight. After cooling to room temperature, the solvent is removed under reduced pressure and the crude product is purified by column chromatography (silica gel, MeOH/EtOAc = 0 ---> 3%) to give a pale brown oil.

**Yield:** 2.01 g (91%); **Raman** (100 mW, 298 K, in $cm^{-1}$): 3072 (14), 2986 (13), 2975 (12), 2941 (25), 2898 (23), 2833 (30), 1667 (14), 1596 (20), 1578 (42), 1476 (11), 1435 (20), 1305 (16), 1284 (12), 1249 (17), 1150 (7), 1118 (6), 1099 (8), 1052 (18), 993 (46), 873 (10), 826 (5), 617 (6), 78 (100); **IR** (ATR, 298 K, in $cm^{-1}$): 3290 (vw), 2938 (vw), 2875 (w), 2815 (vw), 1666 (s), 1576 (m), 1528 (s), 1454 (w), 1431 (vs), 1397 (w), 1362 (w), 1300 (s), 1264 (w), 1220 (s), 1199 (m), 1098 (vs), 965 (w), 920 (w), 875 (m), 849 (m), 776 (vs), 753 (m), 696 (w), 618 (m), 562 (w), 518 (m), 486 (vw), 410 (vw); **$^1$H NMR** ($CDCl_3$, 400 MHz, 300 K, in ppm): $\delta$ = 8.87 (1H, s, H7), 8.04 (1H, d, $^3J_{HH}$ = 4.84 Hz, H11), 7.74 (1H, d, $^3J_{HH}$ = 8.44 Hz, H9), 7.42 (1H, t, $^3J_{HH}$ = 7.72 Hz, H12), 6.70 (1H, t, $^3J_{HH}$ = 6.12 Hz, H10), 3.57 (4H, t, $^3J_{HH}$ = 4.64 Hz, H2 or H3), 3.50 (4H, m, H3 or H2), 3.46 (8H, m, H4 and H5), 3.20 (6H, s, H1); **$^{13}$C{$^1$H} NMR** ($CDCl_3$, 400 MHz, 300 K, in ppm): $\delta$ = 155.90 (1C, s, C6), 153.35 (1C, s, C8), 147.33 (1C, s, C11), 137.06 (1C, s, C12), 117.17 (1C, s, C10), 112.51 (1C, s, C9), 71.41 (2C, s, C5 or C4), 70.51 (2C, s, C3 or C2), 70.40 (2C, s, C2 or C3), 58.53 (2C, s, C1), 48.82 (2C, s, C4 or C5); **Elemental analysis** for $C_{16}H_{27}N_3O_5$, calculated: C 56.29, H 7.97, N 12.31; found: C 56.45, H 7.62, N 12.32; **ESI-MS** (m/z, [Da/e]): 324.4 $[M+H]^+$ ($ESI^+$).

## Synthesis Example 4

Synthesis of 1,1-bis(2-methoxyethyl)-3-(pyridin-2-yl)urea **(4)**

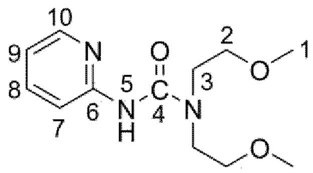

To a suspension of 2-isocyanatopyridine (0.45 g, 3.75 mmol, 1.0 eq.) in 25 ml distilled toluene, bis(2-methoxyethyl)amine (0.50 g, 3.75 mmol, 1.0 eq.) is added dropwise. The suspension is heated to 120 °C and then refluxed overnight. After cooling to room temperature, the solvent is removed under reduced pressure and the crude product is purified by column chromatography (silica gel, MeOH/EtOAc = 0 ---> 3% ---> 6%) to give a pale brown oil.

**Yield:** 0.84 g (88%); **Raman** (100 mW, 298 K, in $cm^{-1}$): 3109 (5), 3072 (19), 2986 (20), 2937 (31), 2898 (27), 2871 (18), 2834 (30), 1668 (14), 1596 (23), 1578 (46), 1477 (14), 1436 (23), 1397 (5), 1305 (15), 1284 (11), 1271 (7), 1249 (16), 1151 (7), 1119 (6), 1099 (7), 1052 (15), 993 (40), 873 (10), 825 (5), 617 (6), 240 (5), 218 (5), 209 (5), 200 (5), 77 (100); **IR** (ATR, 298 K, in $cm^{-1}$): 3298 (vw), 2989 (vw), 2936 (vw), 2880 (vw), 2829 (vw), 1665 (vs), 1576 (s), 1525 (s), 1455 (w), 1430 (vs), 1396 (m), 1364 (w), 1300 (vs), 1266 (w), 1226 (m), 1194 (s), 1148 (w), 1112 (vs), 1070 (m), 1012 (s), 965 (w), 873 (w),

775 (vs), 752 (s), 693 (w), 618 (m), 554 (w), 518 (m), 456 (vw), 410 (w); **$^1$H NMR** ($CDCl_3$, 400 MHz, 300 K, in ppm): δ = 8.81 (1H, s, H5), 8.14 (1H, d, $^3J_{HH}$ = 4.92 Hz, H9), 7.85 (1H, d, $^3J_{HH}$ = 8.48 Hz, H7), 7.51 (1H, t, $^3J_{HH}$ = 6.84 Hz, H10), 6.79 (1H, t, $^3J_{HH}$ = 5.62 Hz, H8), 3.53 (8H, m, H2 and H3), 3.34 (6H, s, H1); **$^{13}$C{$^1$H} NMR** ($CDCl_3$, 400 MHz, 300 K, in ppm): δ = 156.19 (1C, s, C4), 153.58 (1C, s, C6), 147.73 (1C, s, C9), 137.56 (1C, s, C10), 117.53 (1C, s, C8), 112.84 (1C, s, C7), 72.22 (2C, s, C3 or C2), 59.04 (2C, s, C1), 49.09 (2C, s, C2 or C3); **Elemental analysis** for $C_{16}H_{27}N_3O_5$, calculated: C 56.29, H 7.97, N 12.31; found: C 56.45, H 7.62, N 12.32; **ESI-MS** (m/z, [Da/e]): 254.4 $[M+H]^+$ ($ESI^+$).

**Synthesis Example 5**

Synthesis of 1-cyclohexyl-1-(2-(2-methoxyethoxy)ethyl)-3-phenylurea (5)

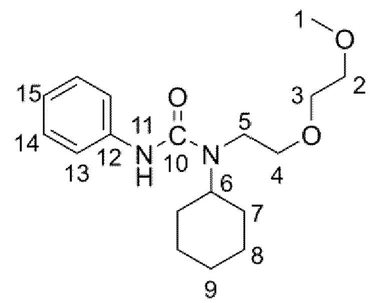

To a 50 mL schlenk flask are added N-(2-(2-methoxyethoxy)ethyl)cyclohexanamine (0.98 g, 4.88 mmol, 1.0 equiv.) and 30 mL dried DCM, this solution is stirred for 30 min. Then phenyl isocyanate (0.58 g, 4.88 mmol, 1.0 equiv.) is dropped to the solution above at -30 °C. The reaction solution is allowed to warm up to room temperature, and then reflux overnight. The reaction is cooled down to room temperature and the solvent is removed under vacuum and the crude product is purified by column chromatography (silica gel, EtOAc/iso-hexane = 0 ---> 11%) to give white solid. Single crystals suitable for X-ray diffraction analysis are obtained by slow evaporation of its DCM solution.

**Yield:** 0.92 g (59%); **Raman** (100 mW, 298 K, in cm$^{-1}$): 3063 (26), 2989 (10), 2975 (8), 2939 (47), 2923 (54), 2853 (36), 2825 (13), 2810 (13), 1659 (27), 1597 (39), 1547 (8), 1476 (7), 1444 (29), 1356 (9), 1306 (24), 1258 (29), 1196 (8), 1179 (7), 1151 (22), 1055 (11), 1030 (27), 1002 (37), 997 (39), 871 (20), 835 (24), 783 (11), 753 (6), 624 (7), 515 (5), 447 (5), 221 (23), 91 (100); **IR** (ATR, 298 K, cm$^{-1}$): 3299 (w), 2973 (vw), 2959 (vw), 2927 (m), 2917 (m), 2851 (w), 2808 (vw), 2360 (vw), 2342 (vw), 1655 (s), 1596 (m), 1550 (s), 1503 (m), 1490 (w), 1473 (w), 1440 (m), 1397 (m), 1378 (vw), 1357 (w), 1307 (m), 1276 (w), 1257 (m), 1232 (s), 1197 (w), 1180 (w), 1133 (m), 1102 (m), 1083 (s), 1054 (m), 1047 (m), 982 (vw), 964 (vw), 929 (vw), 901 (vw), 892 (w), 870 (w), 851 (w), 834 (vw), 822 (vw), 750 (vs), 698 (s), 662 (m), 622 (vw), 606 (vw), 562 (w), 515 (w); **$^1$H NMR** ($CDCl_3$, 400 MHz, 300 K, in ppm): δ = 8.46 (1H, s, H11), 7.41 (2H, d, $^3J_{HH}$ = 7.56 Hz, H13), 7.24 (2H, t, $^3J_{HH}$ = 7.76 Hz, H14), 6.94 (1H, t, $^3J_{HH}$ = 7.46 Hz, H15), 4.15 (1H, tt, $^3J_{HH}$ = 11.66, $^4J_{HH}$ = 3.26 Hz, H6), 3.70 (4H, m, H3 and H4), 3.60 (2H, m, H2), 3.45 (2H, t, $^3J_{HH}$ = 4.40 Hz, H5), 3.38 (3H, s, H1), 1.80 (4H, m, H7), 1.66 (1H, d, $^3J_{HH}$ = 12.96 Hz, H9), 1.35 (4H, sept, $^3J_{HH}$ = 11.84 Hz, H8), 1.07 (1H, qt, $^3J_{HH}$ = 12.78, 3.20 Hz, H9); **$^{13}$C{$^1$H} NMR** ($CDCl_3$, 400 MHz, 300 K, in ppm): δ = 157.22 (1C, s, C10), 140.64 (1C, s, C12), 128.75 (2C, s, C14), 121.89 (1C, s, C15), 119.04 (2C, s, C13), 74.30 (1C, s, C4), 71.78 (1C, s, C2), 71.20 (1C, s, C3), 59.00 (1C, s, C1), 54.73 (1C, s, C6), 43.82 (1C, s, C5), 31.25 (2C, s, C8), 26.05 (2C, s, C7), 25.84 (1C, s, C9); **Elemental analysis** for $C_{18}H_{28}N_2O_3$, calculated: C 67.47, H 8.81, N 8.74; found: C 67.59, H 8.35, N 8.84; **ESI-MS** (m/z, [Da/e]): 321.4 $[M+H]^+$ ($ESI^+$).

**Synthesis Example 6**

Synthesis of 1-cyclohexyl-1-(2-(2-methoxyethoxy)ethyl)-3-(pyridin-2-yl)urea **(6)**

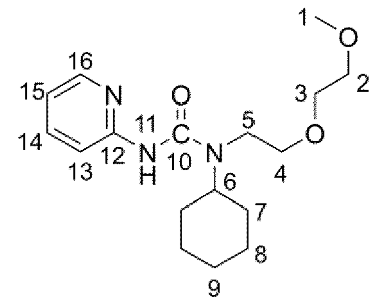

2-isocyanatopyridine (0.94 g, 7.86 mmol, 1.05 equiv.) and 40 mL dried toluene are added to a 100 mL schlenk flask, stir for 30 min. Then N-(2-(2-methoxyethoxy)ethyl)cyclohexanamine is added dropwise. The reaction mixture is heated to 120 °C and then refluxed overnight. The reaction is cooled down to room temperature and the solvent is removed under reduced pressure. The crude product is purified by column chromatography (silica gel, EtOAc/iso-hexane = 0 ---> 10%) to give 6 as pale yellow oil.

**Yield:** 2.01 g (84%); **Raman** (100 mW, 298 K, in $cm^{-1}$): 3108 (6), 3069 (21), 3029 (8), 2939 (85), 2902 (55), 2857 (79), 2821 (25), 1665 (17), 1596 (28), 1578 (48), 1469 (13), 1444 (30), 1350 (7), 1305 (22), 1284 (11), 1263 (15), 1248 (17), 1199 (6), 1153 (9), 1053 (17), 1029 (12), 1001 (16), 987 (36), 875 (12), 840 (14), 784 (10), 622 (7), 222 (11), 200 (6), 76 (100); **IR** (ATR, 298 K, in $cm^{-1}$): 3288 (vw), 2926 (w), 2854 (w), 1661 (vs), 1595 (vw), 1576 (m), 1531 (s), 1453 (w), 1430 (vs), 1396 (m), 1356 (w), 1301 (vs), 1269 (w), 1223 (vs), 1198 (w), 1181 (w), 1148 (w), 1135 (m), 1107 (s), 1054 (m), 1034 (w), 1003 (vw), 985 (w), 962 (vw), 927 (vw), 895 (w), 875 (vw), 849 (vw), 828 (vw), 775 (s), 755 (w), 739 (w), 713 (w), 684 (w), 623 (w), 569 (w), 518 (w), 445 (vw), 410 (w); **$^1$H NMR** ($CDCl_3$, 400 MHz, 300 K, in ppm): δ = 8.95 (1H, s, H11), 8.02 (1H, d, $^3J_{HH}$ = 4.24 Hz, H14), 7.81 (1H, d, $^3J_{HH}$ = 8.32 Hz, H16), 7.40 (1H, t, $^3J_{HH}$ = 7.84 Hz, H13), 6.68 (1H, t, $^3J_{HH}$ = 6.22 Hz, H15), 3.96 (1H, m, H6), 3.52 (6H, m, H2, H3 and H4), 3.30 (2H, t, $^3J_{HH}$ = 4.40 Hz, H5), 3.20 (3H, s, H1), 1.64 (4H, d, $^3J_{HH}$ = 9.08 Hz, H7), 1.49 (1H, d, $^3J_{HH}$ = 12.96 Hz, H9), 1.20 (4H, sext, $^3J_{HH}$ = 11.21 Hz, H8), 0.92 (1H, q, $^3J_{HH}$ = 11.71 Hz, H9); **$^{13}$C {$^1$H} NMR** ($CDCl_3$, 400 MHz, 300 K, in ppm): δ = 155.92 (1C, s, C10), 153.58 (1C, s, C12), 147.29 (1C, s, C14), 137.06 (1C, s, C13), 117.11 (1C, s, C15), 112.58 (1C, s, C16), 73.03 (1C, s, C3 or C2), 71.39 (1C, s, C4), 70.95 (1C, s, C2 or C3), 58.56 (1C, s, C1), 54.45 (1C, s, C6), 43.28 (1C, s, C5), 30.67 (2C, s, C8), 25.59 (2C, s, C7), 25.27 (1C, s, C9); **Elemental analysis** for $C_{17}H_{27}N_3O_3$, calculated: C 63.53, H 8.47, N 13.07; found: C 63.15, H 8.28, N 12.88; **ESI-MS** (m/z, [Da/e]): 322.4 $[M+H]^+$ ($ESI^+$).

**Synthesis of Intermediates**

**Synthesis Example 7**

Synthesis of 2-isocyanatopyridine **(7)**

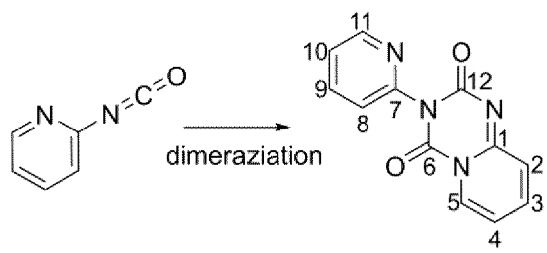


The 2-isocyanatopyridine is synthesized based on the reported procedure (R. Frański, B. Gierczyk, G. Markiewicz and T. Kozik, *Rapid Commun. Mass Spectrom.,* **2015,** 29, 2272-2278). Picolinic acid (4.20 g, 34.12 mmol, 1.0 equiv.) is suspended in 50 ml dried toluene. Diphenylphosphoryl azide (DPPA, 9.86 g, 35.82 mmol, 1.05 equiv.) is added dropwise to the suspension and stirred for 30 min at room temperature. EtsN (5.00 mL, 3.62 g, 35.82 mmol, 1.05 equiv.) is slowly added and a clear pale yellow solution is obtained. The reaction mixture is stirred for 2 h at room temperature and is then heated to 80 °C for 3 h. The reaction mixture is cooled down to room temperature and the solvent is removed under reduced pressure ($N_2$ atmosphere). 10 ml dried DCM are added to wash the residue and is removed again *in vacuo*. This process is repeat 3 times. The sticky residue is filtered off, wash with a mixture of dried $Et_2O$ (11 mL) and dried $CH_3OH$ (4 mL) twice and dried *in vacuo* overnight. The final product is obtained as its dimer 3-(pyridin-2-yl)-2H-pyrido[1,2-a][1,3,5]triazine-2,4(3H)-dione as a pale pink-grey solid.

**Yield:** 3.30 g (80%); **$^1$H NMR** ($CD_2Cl_2$, 500 MHz, 300 K, in ppm): $\delta$ = 8.65 - 8.66 (1H, m, H11), 8.42 - 8.44 (1H, m, H5), 7.93 - 7.97 (1H, m, H3), 7.70 - 7.73 (1H, m, H9), 7.45 - 7.48 (1H, m, H8), 7.38 - 7.39 (1H, m, H4), 7.17 - 7.19 (1H, m, H10), 6.83 - 6.86 (1H, m, H2); **$^{13}$C{$^1$H} NMR** ($CDCl_3$, 400 MHz, 300 K, in ppm): $\delta$ = 113.3 (1C, s, C2), 123.6 (1C, s, C4), 124.4 (1C, s, C10), 124.7 (1C, s, C8), 128.9 (1C, s, C5), 139.1 (1C, s, C3), 141.7 (1C, s, C9), 148.3 (1C, s, C6), 148.4 (1C, s, C7), 150.2 (1C, s, C11), 152.6 (1C, s, C1), 155.0 (1C, s, C12); **ESI-MS** (m/z, [Da/e]): 121.1 [monomer+H]$^+$, 241.1 [dimer+H]$^+$.

### Synthesis Example 8

Synthesis of 2-(2-methoxyethoxy)ethyl 4-methylbenzenesulfonate **(8)**

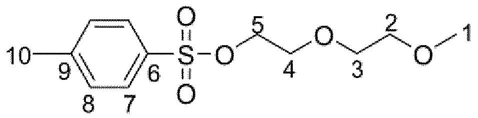

The preparation of **8** is based on a reported procedure (J. Y. Ge, K. Zhang, L. Fan, X. D. Wang, C. H. Zhang, C. Dong, M. S. Wong and S. M. Shuang, Analyst, 2019, 144, 4288-4294). 2-(2-Methoxyethoxy) methanol (4.90 ml, 5.00 g, 41.62 mmol, 1.0 equiv.) is dissolved in a 1:1 mixture of THF and $H_2O$ (15 ml each), and NaOH (3.33 g, 83.25 mmol, 2.0 equiv.) is added slowly with vigorous stirring (exothermic reaction). After cooling to room temperature, the reaction mixture is placed in an ice-water bath to cool down to 0 °C. Then 1.04 equiv. of 4-methylbenzenesulfonyl chloride (8.27 g, 43.38 mmol), dissolved in 20 mL THF, are added dropwise to the mixture at 0 °C and the reaction mixture is stirred for 4 h at room temperature. The reaction mixture is poured into ice water and the resulting mixture is extracted with EtOAc (3 $\times$ 20 mL). The combined organic layer is dried over anhydrous $Na_2SO_4$. The solvent is removed under reduced pressure to give 8.81 g of the product, a colorless oil, which is used for subsequent synthesis without purification.

**Yield:** 8.81 g (77%); **$^1$H NMR** ($CDCl_3$, 400 MHz, 300 K, in ppm): $\delta$ = 7.76 (2H, d, $^3J_{HH}$ = 8.24 Hz, H7), 7.30 (2H, d, $^3J_{HH}$ = 8.08 Hz, H8), 4.13 (2H, t, $^3J_{HH}$ = 4.88 Hz, H5), 3.65 (2H, t, $^3J_{HH}$ = 4.86 Hz, H4), 3.52 - 3.55 (2H, m, H3), 3.43 - 3.45 (2H, m, H2), 3.31 (3H, s, H1), 2.41 (3H, s, H10); **$^{13}$C{$^1$H} NMR** (DMSO-$d_6$, 400 MHz, 300 K, in ppm): $\delta$ = 144.8 (1C, s, C6), 132.5 (1C, s, C9), 130.0 (2C, s, C8), 127.6 (2C, s, C7), 71.1 (1C, s, C2), 69.9 (1C, s, C3), 69.6 (1C, s, C4), 67.9 (1C, s, C5), 58.0 (1C, s, C1), 21.0 (1C, s, C10); **ESI-MS** (m/z, [Da/e]): 275.1 [M+H]$^+$ (ESI$^+$).

### Synthesis Example 9

Synthesis of N-benzyl-2-(2-methoxyethoxy)-N-(2-(2-methoxyethoxy)ethyl)ethan-1-amine **(9)**

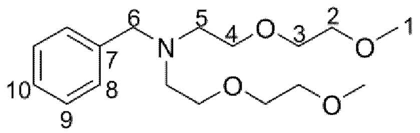

The preparation of **9** is based on a reported procedure (A. Hofmann, C. Graf, S.-H. Kung, M. Kim, X. G. Peng, R. El-Aama, E. Rühl, Synthesis, 2010, 7, 1150-1158). In a 250 mL three-necked flask, $Cs_2CO_3$ (22.35 g, 68.59 mmol, 1.5 equiv.) is added to 100 mL dried MeCN and the mixture is stirred for 20 min. Phenylmethanamine (5.00 mL, 4.90 g, 45.73 mmol, 1.0 equiv.) is added dropwise and the mixture is stirred for another 20 min. Then **8** (25.09 g, 91.46 mmol, 2.0 equiv.) is added and the mixture is refluxed overnight. The reaction mixture is cooled down to room temperature and filtered. The solvent of the filtrate is evaporated, the residue is redissolved in DCM and filtered. The solvent is removed under reduce pressure and the yellow oil is purified by column chromatography (silica gel, acetone/iso-hexane = 0 $\rightarrow$ 25%) to obtain a pale yellow oil.

**Yield:** 9.80 g (69%); **$^1$H NMR** ($CDCl_3$, 400 MHz, 300 K, in ppm): $\delta$ = 7.21 - 7.35 (5H, m, H10, H9, H8 and H7), 3.72 (2H, s, H6), 3.52 - 3.59 (12H, m, H4, H3 and H2), 3.38 (6H, s, H1), 2.78 (4H, t, $^3J_{HH}$ = 6.34 Hz, H5); **$^{13}$C{$^1$H} NMR** (DMSO-$d_6$, 400 MHz, 300 K, in ppm): $\delta$ = 139.9 (1C, s, C7), 128.4 (2C, s, C8), 127.9 (2C, s, C9), 126.6 (1C, s, C10), 71.4 (2C, s, C2), 69.6 (2C, s, C3 or C4), 69.2 (2C, s, C4 or C3), 59.0 (1C, s, C6), 58.0 (2C, s, C1), 53.3 (2C, s, C5); **ESI-MS** (m/z, [Da/e]): 312.0 [M+H]$^+$ (ESI$^+$).

**Synthesis Example 10**

Synthesis of bis(2-(2-methoxyethoxy)ethyl)amine **(10)**

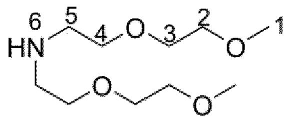

The preparation of **10** is based on a reported procedure (S. Ram and L. D. Spicer, Synth. Commun., 1987, 17, 415-418). Pd/C catalyst (0.8 g) is purged with argon for 0.5 h in a three-necked flask before 50 mL anhydrous MeOH are slowly added followed by ammonium formate (3.85 g, 61.01 mmol, 2.5 equiv.). The mixture is stirred for 10 min until it is completely dissolved and **9** (7.60 g, 24.40 mmol, 1.0 equiv.) is added dropwise. The mixture is refluxed for 4 h, cooled down to room temperature, filtered and the solvent is removed. The residue is washed with DCM, filter and the filtrate is evaporated yielding the pure product **10** as a pale yellow oil.

**Yield:** 5.30 g (98%); **$^1$H NMR** ($CDCl_3$, 400 MHz, 300 K, in ppm): $\delta$ = 3.46 - 3.56 (12H, m, H4, H3 and H2), 3.32 (6H, s, H1), 2.76 (4H, t, $^3J_{HH}$ = 5.34 Hz, H5); **$^{13}$C{$^1$H} NMR** ($CDCl_3$, 400 MHz, 300 K, in ppm): $\delta$ = 71.9 (2C, s, C2), 70.8 (2C, s, C4), 70.3 (2C, s, C3), 59.0 (2C, s, C1), 49.2 (2C, s, C5); **ESI-MS** (m/z, [Da/e]): 222.0 $[M+H]^+$ ($ESI^+$).

**Synthesis Example 11**

Synthesis of (E)-N-cyclohexyl-1-phenylmethanimine **(11)**

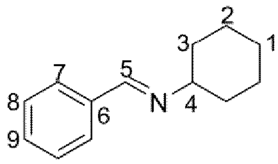

The preparation of **11** is based on a reported procedure (F. Schaufelberger and O. Ramström, Chem. Eur. J., 2015, 21, 12735-12740). Cyclohexanamine (3.50 mL, 3.04 g, 30.60 mmol, 1.0 equiv.) is added to 50 mL anhydrous DCM in the presence of 6 g preactivated 4 Å molecular sieves. To this mixture benzaldehyde (3.11 mL, 3.25 g, 30.60 mmol, 1.0 equiv.) is added under $N_2$ atmosphere and the reaction mixture is refluxed overnight. Upon completion of reaction, the mixture is filtered and concentrated *in vacuo* to obtain pale yellow oily which is used without further purification.

**Yield:** 4.82 g (84%); **$^1$H NMR** ($CDCl_3$, 500 MHz, 300 K, in ppm): $\delta$ = 8.32 (1H, s, H5), 7.73 - 7.76 (2H, m, H7), 7.40 (3H, t, $^3J_{HH}$ = 3.18 Hz, H8 and H9), 3.21 (1H, sept., $^3J_{HH}$ = 4.86 Hz, H4), 1.74 - 1.88 (4H, m, H3), 1.57 - 1.71 (3H, m, H2 and H1), 1.26 - 1.44 (3H, m, H1 and H2); **$^{13}$C{$^1$H} NMR** ($CDCl_3$, 400 MHz, 300 K, in ppm): $\delta$ = 158.5 (1C, s, C5), 136.7 (1C, s, C6), 130.3 (1C, s, C9), 128.5 (2C, s, C7), 128.1 (2C, s, C8), 70.0 (1C, s, C4), 34.4 (2C, s, C3), 25.7 (1C, s, C1), 24.8 (2C, s, C2); **ESI-MS** (m/z, [Da/e]): 187.9 $[M+H]^+$ ($ESI^+$).

**Synthesis Example 12**

Synthesis of N-benzylcyclohexanamine **(12)**

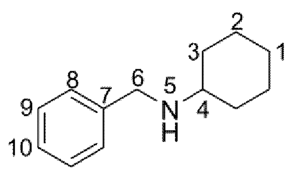

The preparation of **12** is based on a reported procedure (L. Blackburn and R. J. K. Taylor, Org. Lett., 2001, 3,

1637-1639). **11** (2.00 g, 10.68 mmol, 1.0 equiv.) is dissolved in 30 mL anhydrous methanol and the mixture is cooled down to 0 °C. $NaBH_4$ (0.45 g, 11.75 mmol, 1.1 equiv.) is added portion wise and the reaction mixture is stirred for 2 h at room temperature, while the white suspension becomes clear. Then solvent is removed, and the residue is redissolved in 20 mL $Et_2O$. 20 mL saturated $NaHCO_3$ are added to wash the solution. The organic layer is separated, and aqueous phase is extracted with $Et_2O$ (3 × 10 mL). The combined organic phase is dried over anhydrous $Na_2SO_4$. The final product is obtained after filtration and concentrated *in vacuo.*

**Yield:** 1.80 g (82%); **$^1$H NMR** ($CDCl_3$, 400 MHz, 300 K, in ppm): $\delta$ = 7.35 - 7.37 (4H, m, H8 and H9), 7.26 - 7.29 (1H, m, H10), 3.86 (2H, s, H6), 2.54 (1H, t, $^3J_{HH}$ = 10.20 Hz, H4), 1.96 (2H, d, $^3J_{HH}$ = 11.98 Hz, H3), 1.77 - 1.81 (2H, m, H3), 1.64 - 1.68 (1H, m, H5), 1.13 - 1.36 (6H, m, H1 and H2); **$^{13}$C{$^1$H} NMR** ($CDCl_3$, 400 MHz, 300 K, in ppm): $\delta$ = 158.6 (1C, s, C7), 136.7 (1C, s, C10), 130.3 (1C, s, C6), 128.6 (2C, s, C9), 128.1 (2C, s, C8), 70.0 (1C, s, C4), 34.5 (2C, s, C3), 25.8 (1C, s, C1), 24.9 (2C, s, C2); **ESI-MS** (m/z, [Da/e]): 189.9 $[M+H]^+$ ($ESI^+$).

**Synthesis Example 13**

Synthesis of N-benzyl-N-(2-(2-methoxyethoxy)ethyl)cyclohexanamine **(13)**

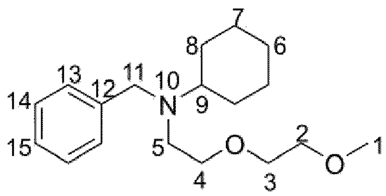

The preparation of **13** is based on a reported procedure (L. Cui, L. W. Ye and L. M. Zhang, Chem. Commun., 2010, 46, 3351-3353). **8** (15.65 g, 57.05 mmol, 1.8 equiv) is added to a mixture of **12** (6.00 g, 31.70 mmol, 1.0 equiv.), NaI (2.38 g, 15.85 mmol, 0.5 equiv.) and $K_2CO_3$ (13.14 g, 95.09 mmol, 3 equiv.) in 60 mL anhydrous $CH_3CN$. The reaction mixture is refluxed for 12 h and cooled down to room temperature before 30 mL DCM are added. The solid is filtered off and the filtrate is concentrated under vacuum. The residue is redissolved in 20 mL DCM and washed 3 times with 20 mL 5% NaOH. The separated organic phase is dried over anhydrous $MgSO_4$ overnight. The final product is obtained as pale yellow oil after filtration and evaporation of the solvent. The product is dried *in vacuo* and used without further purification.

**Yield:** 7.74 g (84%); **$^1$H NMR** ($CDCl_3$, 400 MHz, 300 K, in ppm): $\delta$ = 7.35 (2H, d, $^3J_{HH}$ = 7.28 Hz, H13), 7.27 (2H, t, $^3J_{HH}$ = 7.36 Hz, H14), 7.20 (1H, t, $^3J_{HH}$ = 7.20 Hz, H15), 3.68 (2H, 2, H11), 3.45 - 3.51 (4H, m, H2 and H3), 3.41 (2H, t, $^3J_{HH}$ = 6.96 Hz, H4), 3.35 (3H, s, H1), 2.75 (2H, t, $^3J_{HH}$ = 6.96 Hz, H5), 2.50 (1H, t, $^3J_{HH}$ = 12.66 Hz, H9), 1.85 (2H, d, $^3J_{HH}$ = 11.25 Hz, H8), 1.77 (2H, d, $^3J_{HH}$ = 12.23 Hz, H8), 1.61 (1H, d, $^3J_{HH}$ = 12.43 Hz, H6), 1.04 - 1.17 (5H, m, H6 and H7); **$^{13}$C{$^1$H} NMR** ($CDCl_3$, 400 MHz, 300 K, in ppm): $\delta$ = 141.7 (1C, s, C12), 128.3 (2C, s, C13), 128.1 (2C, s, C14), 126.5 (1C, s, C15), 72.0 (1C, s, C2), 71.3 (1C, s, C3), 70.2 (1C, s, C4), 60.5 (1C, s, C9), 59.0 (1C, s, C11), 55.5 (1C, s, C1), 49.9 (1C, s, C5), 29.1 (2C, s, C8), 26.5 (1C, s, C6), 26.2 (2C, s, C7); **ESI-MS** (m/z, [Da/e]): 292.0 $[M+H]^+$ ($ESI^+$).

**Synthesis Example 14**

Synthesis of N-(2-(2-methoxyethoxy)ethyl)cyclohexanamine **(14)**

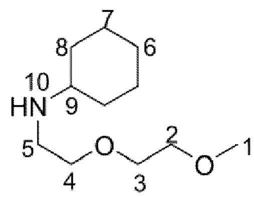

The preparation of **14** is based on a reported procedure (S. Ram and L. D. Spicer, Synth. Commun., 1987, 17, 415-418). Pd/C catalyst (0.4 g) is purged with argon for 0.5 h in a three-necked flask before 30 mL anhydrous MeOH are slowly added followed by ammonium formate (3.35 g, 53.12 mmol, 4.0 equiv.). The mixture is stirred for 10 min until it is completely dissolved and **13** (3.87 g, 13.28 mmol, 1.0 equiv.) is added dropwise. The mixture is refluxed for 4 h, cooled

down to room temperature, filtered, and the solvent is removed. The residue is washed with DCM, filter and the filtrate is evaporated yielding the pure product **14** as a pale yellow oil.

**Yield:** 2.51 g (94%); **$^1$H NMR** ($CDCl_3$, 400 MHz, 300 K, in ppm): $\delta$ = 7.35 (2H, d, $^3J_{HH}$ = 7.28 Hz, H13), 7.27 (2H, t, $^3J_{HH}$ = 7.36 Hz, H14), 7.20 (1H, t, $^3J_{HH}$ = 7.20 Hz, H15), 3.68 (2H, 2, H11), 3.45 - 3.51 (4H, m, H2 and H3), 3.41 (2H, t, $^3J_{HH}$ = 6.96 Hz, H4), 3.35 (3H, s, H1), 2.75 (2H, t, $^3J_{HH}$ = 6.96 Hz, H5), 2.50 (1H, t, $^3J_{HH}$ = 12.66 Hz, H9), 1.85 (2H, d, $^3J_{HH}$ = 11.25 Hz, H8), 1.77 (2H, d, $^3J_{HH}$ = 12.23 Hz, H8), 1.61 (1H, d, $^3J_{HH}$ = 12.43 Hz, H6), 1.04 - 1.17 (5H, m, H6 and H7); **$^{13}$C{$^1$H} NMR** ($CDCl_3$, 400 MHz, 300 K, in ppm): $\delta$ = 141.7 (1C, s, C12), 128.3 (2C, s, C13), 128.1 (2C, s, C14), 126.5 (1C, s, C15), 72.0 (1C, s, C2), 71.3 (1C, s, C3), 70.2 (1C, s, C4), 60.5 (1C, s, C9), 59.0 (1C, s, C11), 55.5 (1C, s, C1), 49.9 (1C, s, C5), 29.1 (2C, s, C8), 26.5 (1C, s, C6), 26.2 (2C, s, C7); **ESI-MS** (m/z, [Da/e]): 201.9 $[M+H]^+$ ($ESI^+$).

The present patent application is supported by the German ministry of economics (BMWK). The support code ("Förderkennzeichen") is 03ETE042C (BMWK; "Verbundvorhaben: SWELL - Stoffliche Wiederverwertung von Elektrolyt-Leitsalzen und -Lösungsmitteln; Teilvorhaben: Recycling und Rückgewinnung des Elektrolytsalzes $LiPF_6$ sowie dessen Zersetzungsprodukte").

## Claims

1. A compound of the following Formula (I),

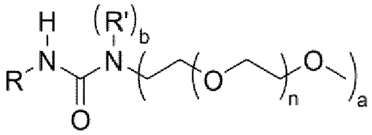

(I),

wherein R is according to the following Formula (II),

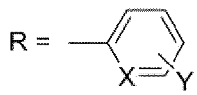

(II),

wherein X is either CH or N,
wherein Y is selected from the group consisting of $CH_3$, $CF_3$, $C_4H_9$, a linear or branched aliphatic alkoxy group comprising from 1 to 12 carbon atoms, $NO_2$, a halogen atom, and an aromatic or heteroaromatic ring comprising 4 to 6 carbon atoms and 0 to 2 nitrogen atoms,
wherein the number of group Y is 0, 1, 2, 3, 4, or, in case X is CH, 5,
wherein, in case of more than one group Y, the groups Y may be the same or different,
wherein R' is either a linear, branched or cyclic aliphatic group comprising 3 to 8 carbon atoms, or is an aromatic group comprising 5 to 14 carbon atoms and 0 to 2 nitrogen atoms in 1, 2, or 3 rings,
wherein n is 0, 1, 2, 3, or 4,
wherein a is 1 or 2,
wherein b is 0 or 1, and
wherein a + b = 2.

2. The compound of Formula (I) according to claim 1, wherein Formula (I) is defined as follows:

X is either CH or N,
Y is selected from the group consisting of $CH_3$, $CF_3$, $C_4H_9$, a linear or branched aliphatic alkoxy group comprising from 1 to 12 carbon atoms, $NO_2$, and a halogen atom,
the number of group Y is 0, 1, 2, or 3,
wherein, in case of more than one group Y, the groups Y are the same,
R' is a linear, branched or cyclic aliphatic group comprising 3 to 8 carbon atoms,
n is 0, 1, or 2,
a is 1 or 2,
b is 0 or 1, and

$$a + b = 2.$$

3. The compound of Formula (I) according to claim 1 or 2, wherein Formula (I) is defined as follows:

X is either CH or N,
Y is selected from the group consisting of $CH_3$, $CF_3$, $C_4H_9$, a linear or branched aliphatic alkoxy group comprising from 1 to 12 carbon atoms, $NO_2$, and a halogen atom,
the number of group Y is 0 or 1,
R' is a linear, branched or cyclic aliphatic group comprising 3 to 8 carbon atoms,
n is 0, 1 or 2,
a is 1 or 2,
b is 0 or 1, and

$$a + b = 2.$$

4. The compound of Formula (I) according to anyone of claims 1 to 3, wherein Formula (I) is defined as follows:

X is either CH or N,
the number of group Y is 0,
R' is a linear, branched or cyclic aliphatic group comprising 3 to 8 carbon atoms,
n is 0, 1 or 2,
a is 1 or 2,
b is 0 or 1, and

$$a + b = 2.$$

5. The compound of Formula (I) according to anyone of claims 1 to 4, wherein Formula (I) is defined as follows:

X is either CH or N,
the number of group Y is 0,
R' is a linear, branched or cyclic aliphatic group comprising of 3 to 6 carbon atoms,
n is 0 or 1,
a is 1 or 2,
b is 0 or 1, and

$$a + b = 2.$$

6. The compound of Formula (I) according to anyone of claims 1 to 5, wherein Formula (I) does not cover the following definitions:

1) X is CH, and Y is $NO_2$, and the number of group Y is 1, and R' is phenyl, and n is 1, and a is 1, and b is 1;
2) R is phenyl, and n is 0, and a is 2, and b is 0; and
3) R is 2-pyridyl, and n is 0, and a is 2, and b is 0.

7. A complex of a compound of Formula (I) according to anyone of claims 1 to 6 with a metal ion.

8. The complex according to claim 7, wherein the metal ion is selected from the group consisting of Co(II) and Ni(II).

9. A solution of a complex according to claim 7 or 8 in a solvent comprising an alcohol of the formula $R^A$-OH, wherein $R^A$ is a linear or branched alkyl residue having from 1 to 6 carbon atoms.

10. The solution according to claim 9, wherein the alcohol of the formula $R^A$-OH is selected from ethanol and 1-butanol.

11. A process of ligand-assisted solid-liquid extraction of metal ions from a mixture comprising a metal salt comprising the following steps:

a) providing a solid mixture comprising a metal salt, preferably comprising a salt of Co(II) and a salt of Ni(II),
b) providing a compound of Formula (I) as defined in anyone of claims 1 to 6,
c) dissolving the compound of Formula (I) in a solvent comprising an alcohol of the formula $R^A$-OH and obtaining a

solution of the compound of Formula (I), wherein $R^A$ is a linear or branched alkyl residue having from 1 to 6 carbon atoms,
d) putting the solid mixture comprising a metal salt of step a) and the solution of step c) together in a container,
e) setting entire container or content of the container in motion, such that all parts of the solid mixture comprising a metal salt of step a) come into contact with the solution of step c),
f) stopping moving the container or the content of the container and allowing separation of a solid phase and a liquid phase from each other in the container,
g) removing the liquid phase from the container.

**12.** The process of ligand-assisted solid-liquid extraction of metal ions from a mixture comprising a metal salt according to claim 11, wherein the alcohol of the formula $R^A$-OH makes up 95 to 100 wt% of the solvent.

**13.** The process of ligand-assisted solid-liquid extraction of metal ions from a mixture comprising a metal salt according to claim 11 or 12, wherein the alcohol of the formula $R^A$-OH is selected from the group consisting of ethanol and 1-butanol.

**14.** The process of ligand-assisted solid-liquid extraction of metal ions from a mixture comprising a metal salt according to anyone of claims 11 to 13, wherein

- the solid mixture comprising a metal salt of step a) comprises sulfate salts of Li(I), Mn(II), Co(II), and Ni(II),
- the process is performed twice, wherein in a subsequent run of the process the solid mixture comprising a metal salt of step a) is obtained from the solid phase referred to in step f) of the process of the previous run of the process,
- in the previous run of the process the solvent is solvent A, which is different from solvent B, which is applied in a subsequent run of the process,
- an alcohol A of the formula $R^A$-OH makes up 99 to 100 wt% of the solvent A, and an alcohol B of the formula $R^A$-OH makes up 99 to 100 wt% of the solvent B, and
- alcohol A and alcohol B are different and are selected from the group consisting of ethanol and 1-butanol, or alcohol A is ethanol and alcohol B is 1-butanol.

**15.** Use of a compound of Formula (I) according to anyone of claims 1 to 6 for extracting metal ions from a mixture comprising a metal salt.

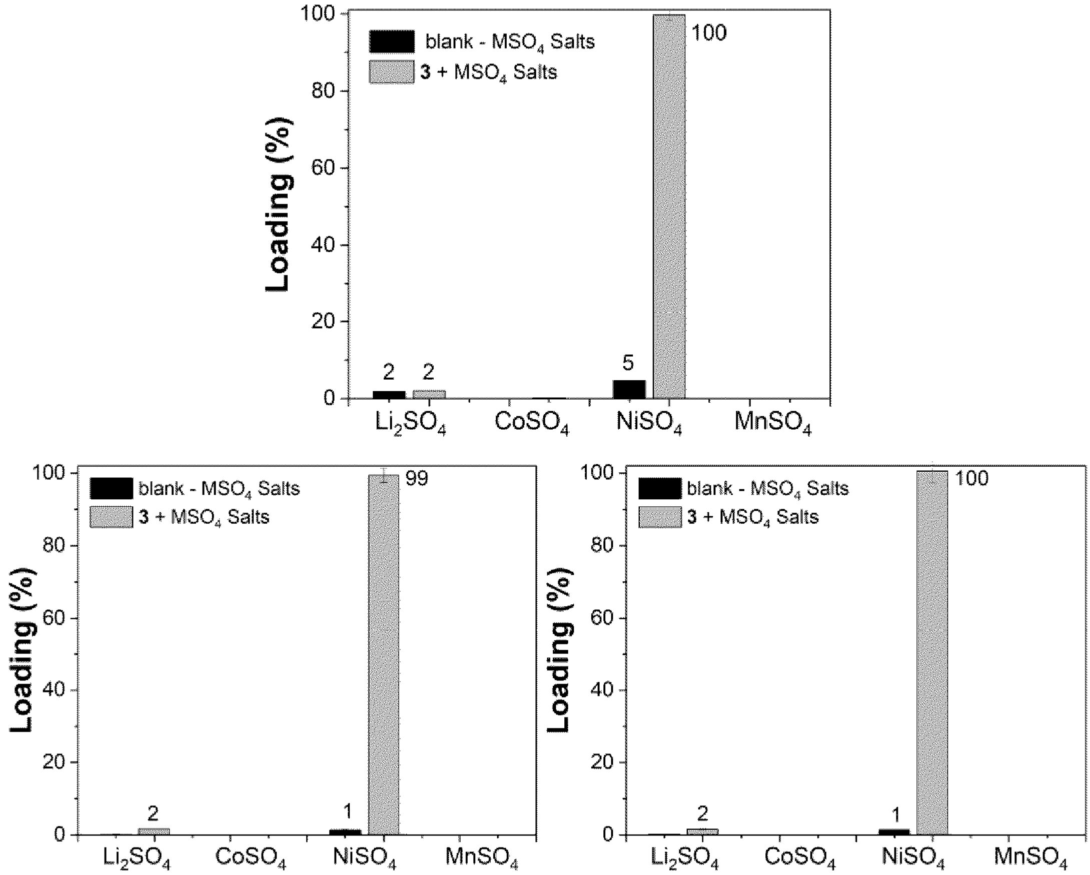


Figure 1: Metal loadings of three independent identical experiments of Extraction Example 1 and of the corresponding reference example.

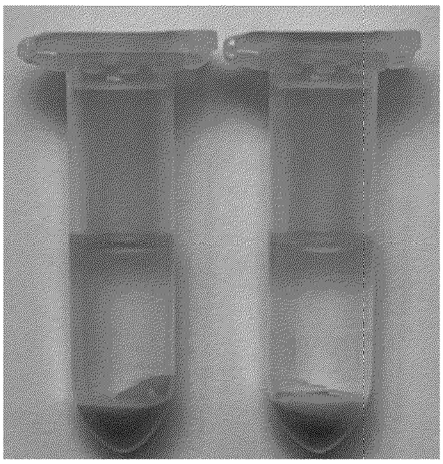

Figure 2: Solution of Extraction Example 1 (left, green color) and of the corresponding reference example (right, colorless).

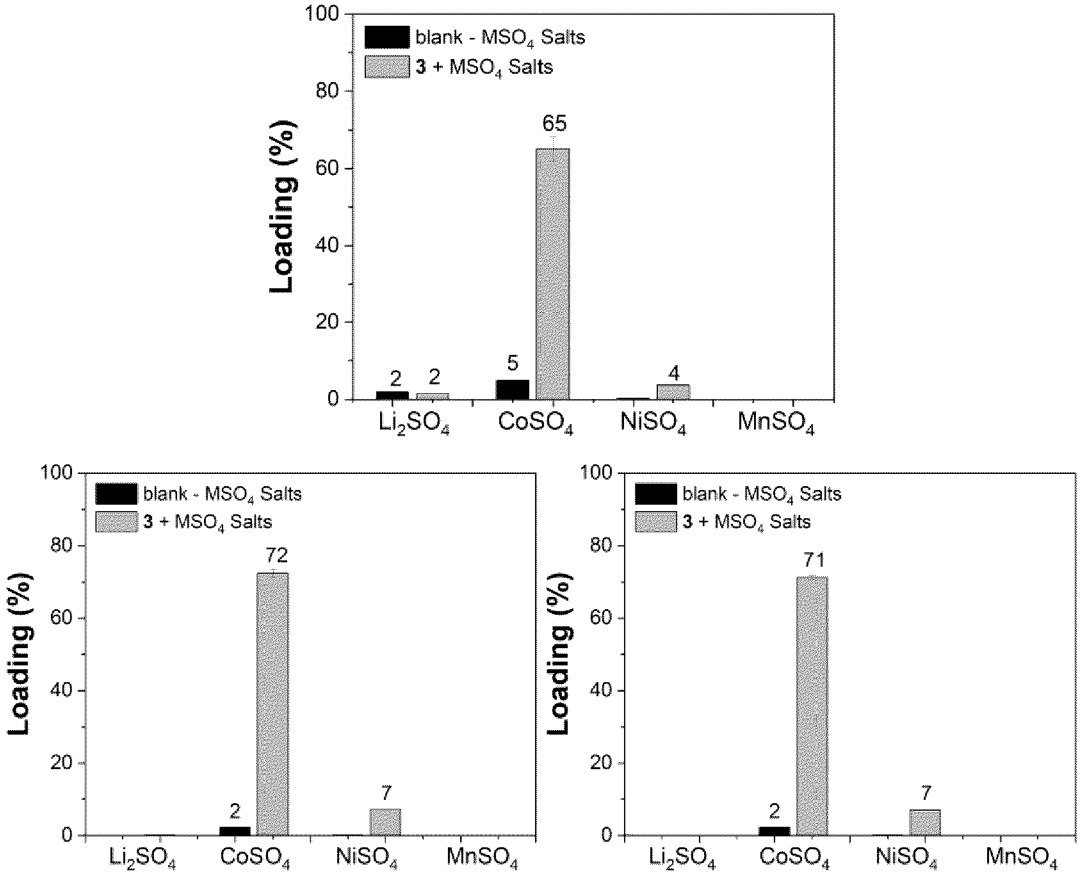


Figure 3: Metal loadings of three independent identical experiments of Extraction Example 2 and of the corresponding reference example.

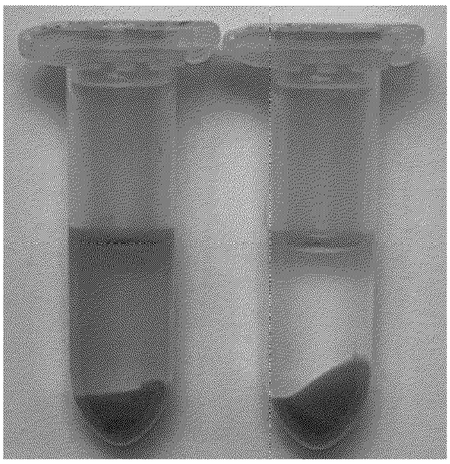

Figure 4: Solution of Extraction Example 2 (left, pink color) and of the corresponding reference example (right, colorless).

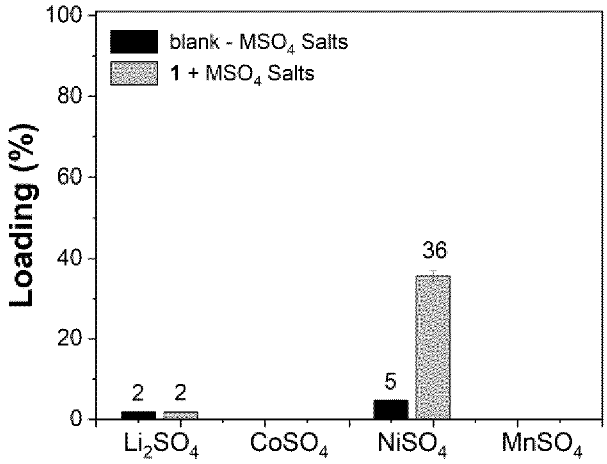


Figure 5: Metal loading of experiment of Extraction Example 3 and of the corresponding reference example.

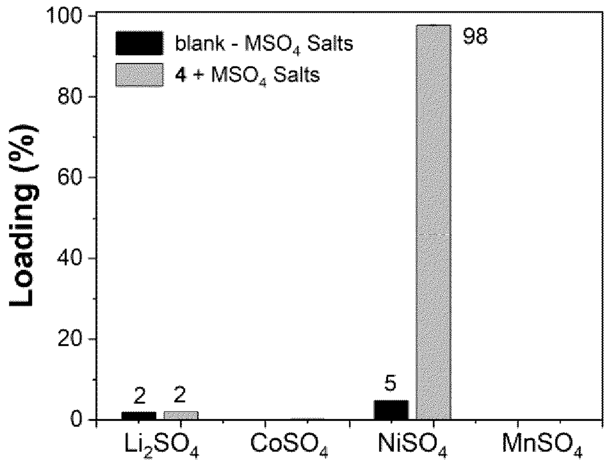


Figure 6: Metal loading of the experiment of Extraction Example 4 and of the corresponding reference example.

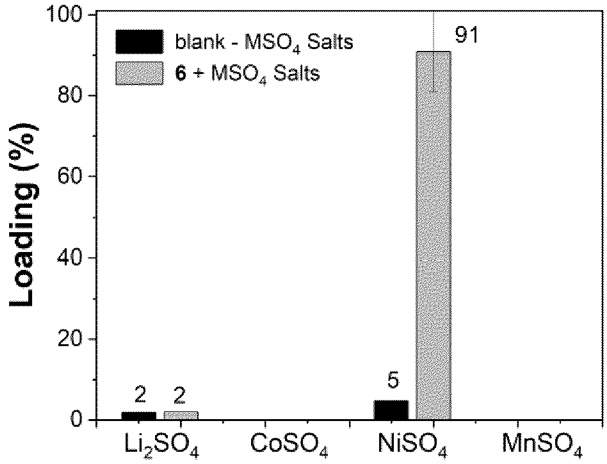


Figure 7: Metal loading of the experiment of Extraction Example 5 and of the corresponding reference example.

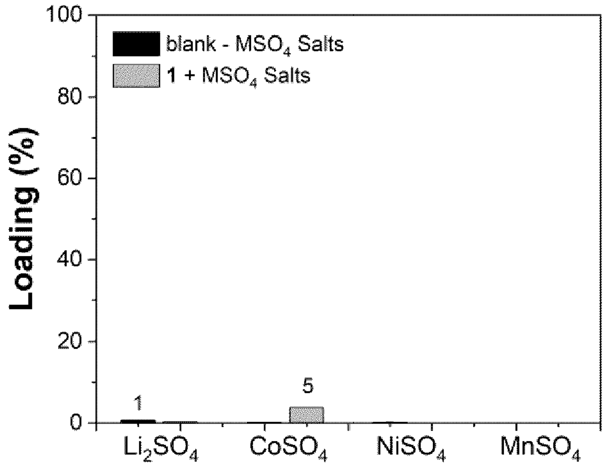


Figure 8: Metal loading of the experiment of Extraction Example 6 and of the corresponding reference example.

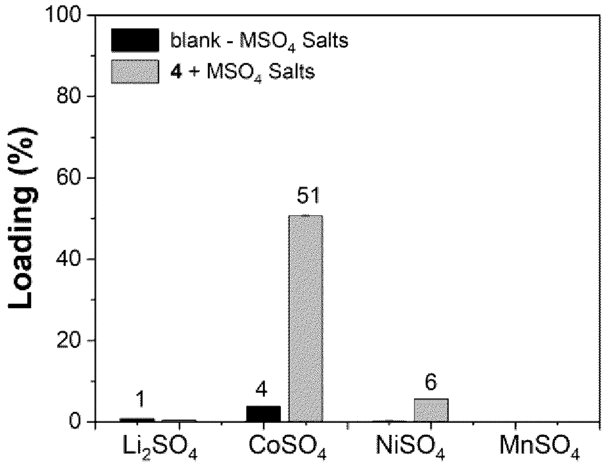


Figure 9: Metal loading of the experiment of Extraction Example 7 and of the corresponding reference example.

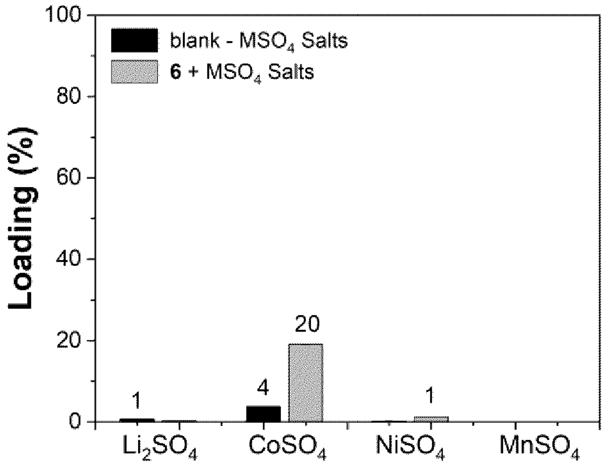


Figure 10: Metal loading of the experiment of Extraction Example 8 and of the corresponding reference example.

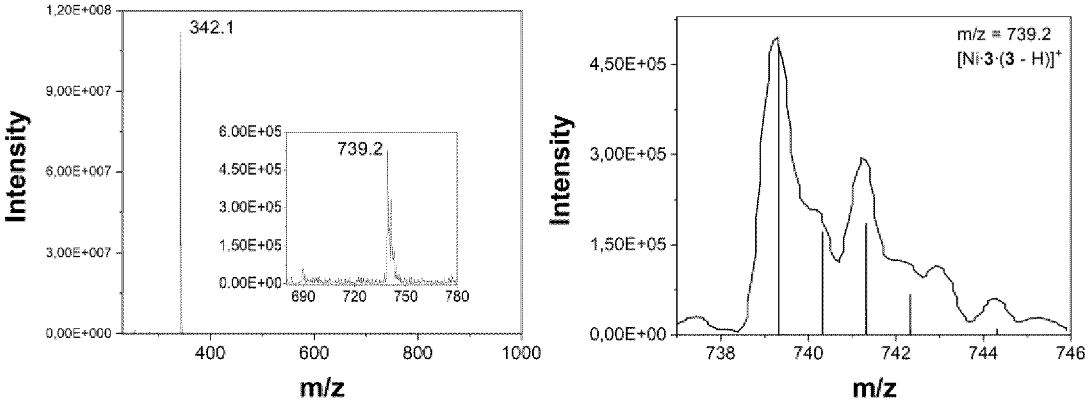


Figure 11: ESI-MS (positive ion mode) of the solution after the solid-liquid extraction using **3** in ethanol of Extraction Example 1 with a magnification of the data obtained between m/z = 680 and 780 (left), signal at m/z = 739.2 with isotope pattern superimposed as vertical lines (right).

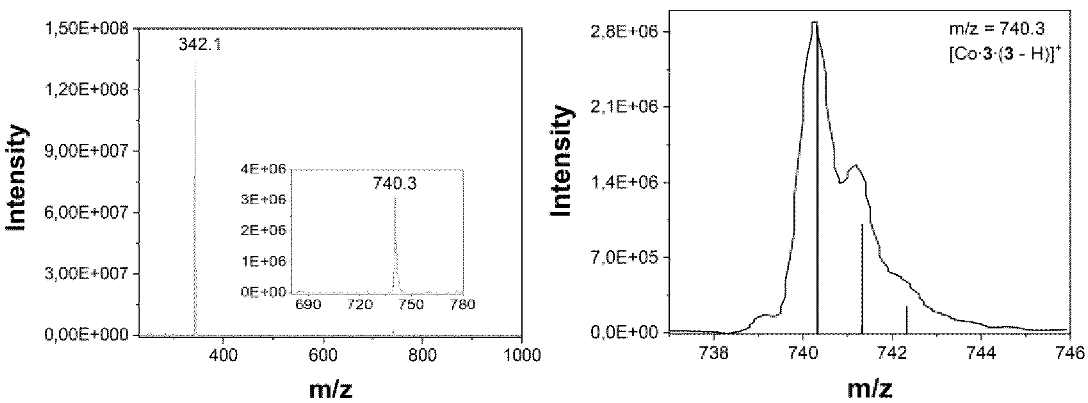


Figure 12: ESI-MS (positive ion mode) of the solution after the solid-liquid extraction using **3** in 1-butanol of Extraction Example 2 with a magnification of the data obtained between m/z = 680 and 780 (left), signal at m/z = 740.3 with isotope pattern superimposed as vertical lines (right).

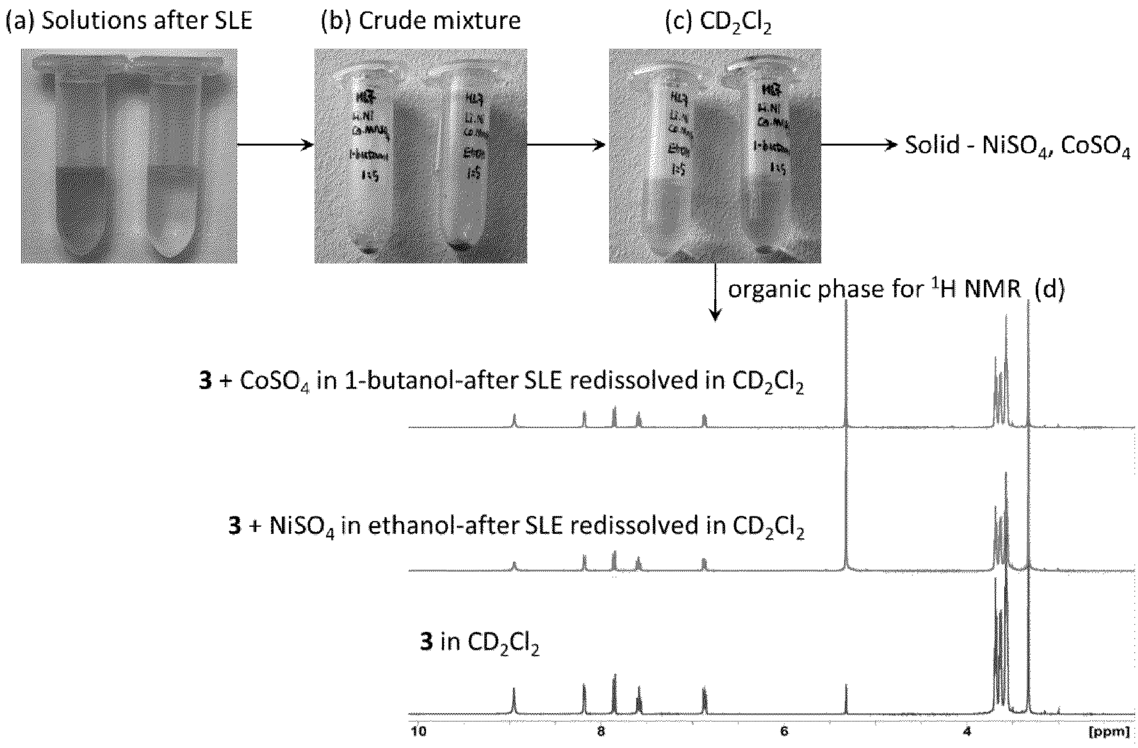


Figure 13: a) Solutions after solid-liquid extraction (SLE) experiments; (b) crude mixtures after solvent evaporation; (c) crude mixtures redissolved in $CD_2Cl_2$; (d) stack of the $^1$H-NMR spectra of **3** and recovered **3** from SLE experiments using ethanol or 1-butanol.

Europäisches Patentamt
European Patent Office
Office européen des brevets

# EUROPEAN SEARCH REPORT

Application Number
EP 24 21 9080

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PÉREZ-CASAS CAROL ET AL: "Detailing Hydrogen Bonding and Deprotonation Equilibria between Anions and Urea/Thiourea Derivatives", THE JOURNAL OF ORGANIC CHEMISTRY, vol. 73, no. 6, 21 February 2008 (2008-02-21), pages 2275-2284, XP093278898, United States ISSN: 0022-3263, DOI: 10.1021/jo702458f Retrieved from the Internet: URL:https://pubs.acs.org/doi/pdf/10.1021/jo702458f> | 1-3,6 | INV. C07D213/75 C07C275/28 C07F15/04 C07F15/06 C22B3/00 C22B23/00 |
| A | * page 2276; compound 2 * | 4,5,7-15 | |
| | ----- | | |
| X | BERNTSSON P ET AL: "Gastric acid secretion inhibitors. I. Compounds chemically related to lidocaine", ACTA PHARMACEUTICA SUECICA, ROYAL PHARMACEUTICAL INSTITUTE, SWEDEN, vol. 13, no. 5-6, 1 January 1976 (1976-01-01), pages 385-390, XP009114493, ISSN: 0001-6675 | 1-3,6 | |
| A | * page 388; compound 29 * | 4,5,7-15 | |
| | ----- | | |
| A | CN 117 186 063 A (UNIV CENTRAL SOUTH) 8 December 2023 (2023-12-08) * examples * * claims * | 1-15 | |
| | ----- | | |
| A | US 2021/376400 A1 (BHAVE RAMESH R [US] ET AL) 2 December 2021 (2021-12-02) * examples * * claims * | 1-15 | |
| | ----- | | |
| | -/-- | | |

| TECHNICAL FIELDS SEARCHED (IPC) |
|---|
| C07D |
| C07F |
| C07C |
| C22B |

The present search report has been drawn up for all claims

1

| Place of search | Date of completion of the search | Examiner |
|---|---|---|
| Munich | 20 May 2025 | Stix-Malaun, Elke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 21 9080

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2024/072238 A1 (ELION SP Z O O [PL]) 4 April 2024 (2024-04-04) * examples * * claims * ----- | 1-15 | |
| A | WANG BAOYING ET AL: "Efficient separation and recovery of cobalt(II) and lithium(I) from spent lithium ion batteries (LIBs) by polymer inclusion membrane electrodialysis (PIMED)", CHEMICAL ENGENEERING JOURNAL, ELSEVIER, AMSTERDAM, NL, vol. 430, 13 October 2021 (2021-10-13), XP086890350, ISSN: 1385-8947, DOI: 10.1016/J.CEJ.2021.132924 [retrieved on 2021-10-13] * abstract * * figures * * examples * ----- | 1-15 | |
| | | | TECHNICAL FIELDS SEARCHED (IPC) |

1

The present search report has been drawn up for all claims

| Place of search | Date of completion of the search | Examiner |
|---|---|---|
| Munich | 20 May 2025 | Stix-Malaun, Elke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO.

EP 24 21 9080

This annex lists the patent family members relating to the patent documents cited in the above-mentioned European search report. The members are as contained in the European Patent Office EDP file on
The European Patent Office is in no way liable for these particulars which are merely given for the purpose of information.

20-05-2025

| Patent document cited in search report | Publication date | Patent family member(s) | Publication date |
|---|---|---|---|
| CN 117186063 A | 08-12-2023 | NONE | |
| US 2021376400 A1 | 02-12-2021 | AU 2021282007 A1 | 05-01-2023 |
| | | BR 112022023627 A2 | 20-12-2022 |
| | | CA 3179484 A1 | 02-12-2021 |
| | | CN 115697578 A | 03-02-2023 |
| | | EP 4157558 A1 | 05-04-2023 |
| | | JP 7620647 B2 | 23-01-2025 |
| | | JP 2023527386 A | 28-06-2023 |
| | | KR 20230016205 A | 01-02-2023 |
| | | US 2021376400 A1 | 02-12-2021 |
| | | WO 2021242429 A1 | 02-12-2021 |
| WO 2024072238 A1 | 04-04-2024 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Solvent Extraction Principles and Practice, Revised and Expanded. CRC Press, 2004
- **P. A. TASKER** ; **E. D. DOIDGE**. Compr. Coord. Chem.. Elsevier, 2021, vol. IIII, 494-557
- **C. Y. CHENG** ; **K. R. BARNARD** ; **W. ZHANG** ; **D. J. ROBINSON**. *Solvent Extr. Ion Exch.*, 2011, vol. 29, 719-754
- **J. Y. GE** ; **K. ZHANG** ; **L. FAN** ; **X. D. WANG** ; **C. H. ZHANG** ; **C. DONG** ; **M. S. WONG** ; **S. M. SHUANG**. *Analyst*, 2019, vol. 144, 4288-4294
- **A. HOFMANN** ; **C. GRAF** ; **S.-H. KUNG** ; **M. KIM** ; **X. G. PENG** ; **R. EL-AAMA** ; **E. RÜHL**. *Synthesis*, 2010, vol. 7, 1150-1158
- **S. RAM** ; **L. D. SPICER**. *Synth. Commun*, 1987, vol. 17, 415-418
- **F. SCHAUFELBERGER** ; **O. RAMSTRÖM**. *Chem. Eur. J.*, 2015, vol. 21, 12735-12740
- **L. BLACKBURN** ; **R. J. K. TAYLOR**. *Org. Lett.*, 2001, vol. 3, 1637-1639
- **L. CUI** ; **L. W. YE** ; **L. M. ZHANG**. *Chem. Commun.*, 2010, vol. 46, 3351-3353
- **S. RAM** ; **L. D. SPICER**. *Synth. Commun.*, 1987, vol. 17, 415-418